# EUROPÄISCHE PATENTSCHRIFT

(11) **EP 2 692 376 B1**
(45) Veröffentlichungstag und Bekanntmachung des Hinweises auf die Patenterteilung: **03.04.2019**
(21) Anmeldenummer: 12178913.5
(22) Anmeldetag: 01.08.2012
(51) Int. Cl.: A61M 5/20, A61M 5/315, A61M 5/31

(54) **Injektionsvorrichtung mit einer helix- bzw. wendelförmigen Dosisskala**
Injection device with helical or spiral dose scale
Dispositif d'injection avec échelle de dosage en forme d'hélice ou de spirale

(43) Veröffentlichungstag der Anmeldung: 05.02.2014
(73) Patentinhaber: TecPharma Licensing AG, 3401 Burgdorf (CH)
(72) Erfinder: Streit, Ursina, 3322 Schönbühl (CH); Hirschel, Jürg, 3007 Bern (CH); Eich, Adrian, 3374 Wangenried (CH)

(56) Entgegenhaltungen:
- WO-A1-2005/018721
- WO-A1-2005/046770
- WO-A1-2010/140974
- WO-A1-2011/060785
- WO-A2-2010/089417

## Beschreibung

Die Erfindung betrifft eine Antriebs- und Dosiervorrichtung für eine Injektionsvorrichtung zur Verabreichung eines flüssigen Produkts, insbesondere eines Medikaments, wie zum Beispiel Insulin. Mit der Antriebs- und Dosiervorrichtung ist eine zu verabreichende Produktdosis einstellbar und vorzugsweise ausschüttbar, wobei diese Schritte mehrfach wiederholt werden können. Die Erfindung betrifft somit auch eine Injektionsvorrichtung mit einer solchen Antriebs- und Dosiervorrichtung.

Aus dem Stand der Technik, insbesondere der WO 2009/105909 A1 ist eine Injektionsvorrichtung bekannt, die ein Gehäuse aufweist, in der eine Dosisanzeigehülse über deren Umfang eine wendelförmige Skala angeordnet ist, aufgenommen ist. Am hinteren Ende des Gehäuses ist ein Dosierknopf im Bezug auf das Gehäuse drehbar und axialfest angeordnet. Durch Drehung des Dosierknopfs wird die Dosisanzeigetrommel an einem am Gehäuse gebildeten Gewinde entlang geschraubt. Das Gewinde weist die gleiche Steigung wie die wendelförmige Skala am Umfang des Dosisanzeigeelements auf.

Die WO 2010/140974 A1 und WO 2005/018721 A1 beschreiben ein Injektionsgerät mit einem Einstellglied, das an seinem distalen Ende einen Einstellknopf aufweist, der zur Einstellung der Injektionsdosis dient. Das Injektionsgerät weist ferner ein Dosisanzeigeelement mit einer wendelförmigen Dosisskala auf der Aussenseite auf. Das Dosisanzeigeelement ist mit einem Aussengewinde in einem Gewindeeingriff mit dem Gehäuse und mit einem Innengewinde in einem Gewindeeingriff mit einer Antriebshülse, der in Bezug auf das Gehäuse verdrehgesichert ist aber axial verschiebbar ist. Das Gewinde für den Gewindeeingriff mit dem Gehäuse weist eine gleich große Steigung wie die wendelförmige Dosisskala auf.

Die WO 2011/060785 beschreibt ein Injektionsgerät mit einem Einstellglied, das an seinem distalen Ende einen Einstellknopf aufweist, der zur Einstellung der Injektionsdosis dient. Das Injektionsgerät weist ferner ein Dosisanzeigeelement mit einer wendelförmigen Dosisskala auf der Aussenseite auf. Das Dosisanzeigeelement ist mit einem Antriebsglied rotational verbunden wobei das Antriebsglied in einem Gewindeeingriff mit einer Kolbenstange ist. Das Gewinde für den Gewindeeingriff mit der Kolbenstange weist eine gleich große Steigung wie die wendelförmige Dosisskala auf.

Die WO 2010/089417 beschreibt ein Injektionsgerät mit einem Einstellglied, das an seinem distalen Ende einen Einstellknopf aufweist, der zur Einstellung der Injektionsdosis dient. Das Injektionsgerät weist ferner eine wendelförmige Dosisskala auf. Die Dosisskala ist mit einem Antriebsglied rotational verbunden wobei das Antriebsglied in einem Gewindeeingriff mit einer Mutter ist. Das Gewinde für den Gewindeeingriff mit der Mutter weist eine gleich große Steigung wie die wendelförmige Dosisskala auf.

Die WO 2005/046770 A1 beschreibt ein Injektionsgerät mit einem Einstellglied, das an seinem distalen Ende einen Einstellknopf aufweist, der zur Einstellung der Injektionsdosis dient. Das Einstellglied weist ferner eine wendelförmige Dosisskala auf. Das Einstellglied ist mit einem ersten Gewinde in einem Gewindeeingriff mit dem Gehäuse und mit einem zweiten Gewinde in einem Gewindeeingriff mit einem Schieber, der im Bezug auf das Gehäuse verdrehgesichert ist aber axial verschiebbar ist. Das Gewinde für den Gewindeeingriff mit dem Gehäuse weist eine gleich große Steigung wie die wendelförmige Dosisskala auf, während das Gewinde für den Eingriff mit dem Schieber eine kleinere Steigung aufweist als die Dosisskala.

Die aus dem Stand der Technik bekannten Vorrichtungen sind durchaus anspruchsvoll hinsichtlich ihrer Endmontage. Insbesondere bei der WO 2005/046770 A1 ist es erforderlich, dass das Einstellglied während der Montage mit zwei Gewinden unterschiedlicher Steigung in einen Eingriff gebracht wird.

Es ist eine Aufgabe der Erfindung, eine Antriebs- und Dosiervorrichtung anzugeben, welche einfach zu montieren und somit kostengünstig in der Herstellung ist.

Die Aufgabe wird mit der Antriebs- und Dosiervorrichtung nach Anspruch 1 gelöst. Vorteilhafte Weiterentwicklungen ergeben sich aus den abhängigen Ansprüchen, der Beschreibung und den Figuren.

Die Erfindung geht von einem Antriebsmechanismus, insbesondere einer Antriebs- und Dosiervorrichtung für eine Injektionsvorrichtung zum Verabreichen eines flüssigen Medikaments oder Produkts aus. Die Antriebs- und Dosiervorrichtung weist vorteilhaft ein Gehäuse auf. Das Gehäuse ist vorzugsweise hülsenförmig und/oder länglich ausgebildet. Das Gehäuse kann sich zum Beispiel entlang seiner Längsachse erstrecken.

Das Gehäuse kann optional einen Produktbehälter aufnehmen oder selbst den Produktbehälter bilden. Das Gehäuse kann ein- oder mehrteilig sein. Zum Beispiel kann das Gehäuse einen proximalen Gehäuseteil bilden der die Antriebs- und Dosiervorrichtung umfasst oder aufweist. Das Gehäuse kann ferner einen Produktbehälterhalter aufweisen, der den Produktbehälter, wie zum Beispiel eine Karpule, aufnimmt und mit dem Gehäuse oder dem proximalen Gehäuseteil verbunden ist. Diese Verbindung kann derart sein, dass der Produktbehälter und das Gehäuse oder der proximale Gehäuseteil nach dem Verbinden unlösbar, das heißt nur durch Zerstörung von Verbindungselementen voneinander lösbar sind. Eine solche Lösung ist insbesondere bei Einweginjektionsvorrichtungen von Vorteil, die, nach dem das in dem Produktbehälter enthaltene Produkt vollständig ausgeschüttet ist, als ganzes entsorgt werden. Alternativ kann der Produktbehälterhalter auch lösbar an dem Gehäuse befestigt sein, wodurch es möglich ist, wenngleich auch weniger bevorzugt, die Antriebs- und Dosiervorrichtung gegebenenfalls mehrfach zu verwenden, das heißt einen leeren Produktbehälter gegen einen gefüllten Produktbehälter auszutauschen.

Das Gehäuse dient vorzugsweise dazu, vom Verwender der Vorrichtung ergriffen zu werden. Insbesondere kann das Gehäuse eine im Wesentlichen zylindrische Form aufweisen. Das Gehäuse kann zum Beispiel eine Zeigeeinrichtung, insbesondere ein Fenster aufweisen, mittels der oder durch welches eine aktuell eingesteckte Dosis, vorzugsweise von einer Skala eines Dosiseinstellelements, ablesbar ist.

Die Antriebs- und Dosiervorrichtung, die zusammen mit dem Produktbehälter eine Injektionsvorrichtung bildet, umfasst ein Dosisanzeigeelement, welches zum Beispiel in dem Gehäuse angeordnet sein kann. Über den Umfang, des vorzugsweise hülsenförmigen Dosisanzeigeelements ist eine wendelförmige oder helixförmige Dosisskala angeordnet. Ein solches Dosisanzeigeelement wird üblicherweise auch als Dosisanzeigetrommel bezeichnet. Die Dosisskala kann sich über den Umfang des Dosisanzeigeelements wendel- oder helixförmig erstrecken, das heißt, dass die Dosisskala mehrfach über den Umfang des Dosisanzeigeelements umlaufen kann. Die Dosisskala umfasst vorzugsweise eine Vielzahl von Werten, insbesondere Zahlenwerten, die aneinandergereiht angeordnet sind und die Dosisskala ergeben. Die Werte können somit wendel- oder helixförmig aneinandergereiht sein, so dass sich eine wendel- oder helixförmige Dosisskala ergibt. Vorzugsweise handelt es sich um Zahlenwerte, welche die auszuschüttende Produktdosis in internationalen Einheiten (IU) angeben.

Die wendel- oder helixförmige Skala windet sich mit einer Steigung, vorzugsweise einer konstanten Steigung über den Umfang des Dosisanzeigeelements. In Anlehnung an die für Gewinde definierte Gewindesteigung wird als Steigung der Dosisskala der Weg entlang der Längsachse, welchen die Skala bei einer vollen Umdrehung zurücklegt, verstanden. Vorteile einer wendelförmigen Steigung sind, dass das Dosisanzeigeelement mit mehr als einer Umdrehung gedreht werden kann, ohne dass sich die Skalenwerte wiederholen, wodurch vorteilhaft die Skalenwerte größer oder mehr Skalenwerte dargestellt werden können.

Die Antriebs- und Dosiervorrichtung umfasst ferner eine Zeigeeinrichtung und ein vom Verwender der Antriebs- und Dosiervorrichtung greifbares Dosierglied. Zur Einstellung der zu verabreichenden Dosis ist mittels Drehung des Dosierglied relativ zu der Zeigeeinrichtung das Dosisanzeigeelement relativ zu der Zeigeeinrichtung um eine Drehachse, die der genannten Längsachse entspricht, schraubbar und relativ zu dem Dosierglied und/oder einem Betätigungsglied entlang der Drehachse bewegbar, insbesondere axial bewegbar. Das vorzugsweise hülsenförmige Dosierglied kann zum Beispiel am hinteren oder proximalen Ende des Gehäuses oder der Antriebs- und Dosiervorrichtung angeordnet sein. Das Dosierglied kann zum Beispiel axialfest und drehbar mit dem Gehäuse oder einem gehäusefesten Element verbunden oder daran befestigt sein.

Das Dosierglied kann als Dosierknopf ausgebildet sein und optional als Dosiseinstellglied bezeichnet werden. Das Dosierglied ist vorzugsweise vom Verwender (Patient, Arzt, medizinisches Hilfspersonal) der Antriebs- und Dosiervorrichtung greifbar und bildet vorzugsweise eine äußere, insbesondere von außen zugängliche Oberfläche der Antriebs- und Dosiervorrichtung. Zur Einstellung der auszuschüttenden oder zu verabreichenden Dosis wird das Dosierglied vorzugsweise vom Verwender ergriffen und relativ zu dem Gehäuse und insbesondere der Zeigeeinrichtung um die Drehachse, die vorzugsweise der Längsachse der zum Beispiel länglich ausgestalteten Antriebs- und Dosiervorrichtung entspricht, verdreht. Das vorzugsweise axialfest und drehbar mit dem Gehäuse verbundene Dosierglied ermöglicht eine intuitive Handhabung der Vorrichtung durch den Verwender, der lediglich eine Drehbewegung des Dosierglieds zur Dosiseinstellung ausführen braucht.

Mittels der Zeigeeinrichtung, die bevorzugt an dem Gehäuse gebildet wird, ist ein Wert der Dosisskala ablesbar, welcher der eingestellten Dosis entspricht. Die Zeigeeinrichtung kann zum Beispiel ein Fenster sein, das durch einen Durchbruch im Gehäuse oder durch einen transparenten Einsatz gebildet sein kann. Alternativ oder optional kann die Zeigeeinrichtung ein Pfeil sein oder einen Pfeil aufweisen, der zum Beispiel zusätzlich zu dem Fenster den Wert der Dosisskala markiert, welcher der eingestellten Dosis entspricht. Dies ist zum Beispiel dann von Vorteil, wenn in dem Fenster noch ein anderer Wert zumindest teilweise erscheint, um eine eindeutige Dosisauswahl sicherzustellen. Der Zeiger kann zum Beispiel ein Vorsprung oder ein oder eine Kerbe oder dergleichen sein.

Für die intuitive Bedienung ist vorteilhaft, wenn das Dosisanzeigeelement bei Drehung des Dosierglieds um einen Drehwinkel ebenfalls um diesen Drehwinkel verdreht wird.

Die Antriebs- und Dosiervorrichtung kann ein Betätigungsglied zum Beispiel in der Gestalt eines Betätigungsknopfs aufweisen. Das Betätigungsglied kann eine äußere Oberfläche der Antriebs- und Dosiervorrichtung bilden und/oder von außen zugänglich sein. Das Betätigungsglied kann am proximalen, insbesondere hinteren Ende der Antriebs- und Dosiervorrichtung gebildet sein oder dieses Ende bilden. Das Betätigungsglied lässt sich auf diese Weise vorteilhaft mit dem Daumen der Hand, die das Gehäuse umgreift, betätigen, insbesondere drücken. Durch Loslassen des Betätigungsglieds kann die Betätigung beendet werden. Unter "betätigen" wird die Verschiebung des Betätigungsglieds in die Antriebs- und Dosiervorrichtung, insbesondere in distale Richtung verstanden, wodurch insbesondere eine Produktausschüttung bewirkt werden kann. Das Betätigungsglied ist vorteilhaft relativ zu dem Dosierglied verschiebbar, insbesondere um einen Betätigungshub, und kann insbesondere von dem Dosierglied axial verschiebbar aufgenommen sein. Vorzugsweise ist das Betätigungsglied von der Axialbewegung des Dosisanzeigeelements, die es während der Dosiseinstellung relativ zu dem Dosierglied ausführt, entkoppelt. Mit anderen Worten, behält das Betätigungsglied seine Position entlang der Längsachse im Bezug auf das Dosierglied bei, wenn das Dosierglied gedreht wird.

Das Betätigungsglied kann vorteilhaft gegen die Kraft mindestens einer Feder, insbesondere einer Rücksetz- oder Kupplungsfeder verschiebbar, insbesondere betätigbar sein, wodurch diese mindestens eine Feder gespannt wird. Durch Loslassen kann diese Feder das Betätigungsglied zurücksetzen, insbesondere relativ zu dem Dosierglied, insbesondere in proximale Richtung oder aus der Antriebs- und Dosiervorrichtung heraus, verschieben. Die Rücksetzfeder kann zwischen dem Betätigungsglied und dem Dosierglied angeordnet sein und sich zum Beispiel daran abstützen.

Die Antriebs- und Dosiervorrichtung umfasst ferner ein Lagerelement, mit dem das Dosisanzeigeelement in einem Gewindeeingriff ist. Das Dosisanzeigeelement weist ein Gewinde auf, welches in ein Gewinde des Lagerelements eingreift. Das Lagerelement kann zum Beispiel ein Außengewinde und das Dosisanzeigeelement kann zum Beispiel ein Innengewinde aufweisen, oder umgekehrt. Dieser Eingriff bewirkt, dass das Dosisanzeigeelement relativ zu dem Lagerelement schraubbar ist. Es ist von Vorteil, dass das Lagerelement drehfest mit dem Gehäuse verbunden ist oder in einem drehfesten Eingriff mit dem Gehäuse steht, wobei bevorzugt ist, dass das Lagerelement relativ zu dem Gehäuse axial verschiebbar ist. Insbesondere kann das Lagerelement durch Drehen des Dosierglieds axial verschoben werden.

Die Gewindesteigung des Gewindes des Dosisanzeigeelements und somit auch des Gewindes des Lagerelements ist ungleich der Steigung der wendelförmigen Skala, insbesondere größer oder kleiner als die Steigung der wendelförmigen Skala. Die unterschiedlichen Steigungen ermöglichen es, das Lagerelement an einem Teil anzubringen, welches bei der Dosiseinstellung zumindest eine Axialbewegung entlang der Längsachse ausführt, wobei das Dosisanzeigeelement vorteilhaft ohne ein in das Gehäuse eingreifendes Gewinde auskommen kann. Dies erleichtert die Montage der Antriebs- und Dosiervorrichtung und senkt somit die Kosten.

Während der Dosiseinstellung oder der Drehung des Dosierglieds führt das Dosisanzeigeelement - trotz der genannten unterschiedlichen Steigungen - eine Schraubbewegung relativ zu dem Gehäuse oder der Zeigeeinrichtung aus, die gleich der Steigung der Dosisskala ist. Hierdurch wird sichergestellt, dass zumindest ein Skalenwert der Dosisskala ordnungsgemäß in der Zeigeeinrichtung ablesbar ist. Das Teil, an dem das Lagerelement befestigt ist und das relativ zu dem Lagerelement drehbar und axialfest ist, wird bei der Drehung des Dosierglieds vorzugsweise um einen Weg entlang der Längsachse verschoben, welcher der Differenz aus der Steigung der Dosisskala und der Steigung des Gewindeeingriffs zwischen Lagerelement und Dosisanzeigeelement entspricht.

Vorzugsweise ist eine Gewindehülse drehbar und axialfest mit dem Lagerelement verbunden.

Die Gewindehülse kann ein Gewinde, insbesondere ein Innengewinde, welches in ein Gewinde, insbesondere Außengewinde, eines Vortriebsglieds, wie zum Beispiel einer Kolbenstange, eingreift, aufweisen.

Alternativ oder zusätzlich kann die Gewindehülse ein Gewinde, insbesondere ein zweites Gewinde, welches vorzugsweise ein Außengewinde ist, aufweisen, wobei das zweite Gewinde in ein gehäusefestes Gewinde eingreift, insbesondere in ein Gewinde eines mit dem Gehäuse fest verbundenen Teils oder in das Gehäuse direkt. Zur besseren Unterscheidbarkeit der Gewinde kann das in das Gewinde des Vortriebsglieds eingreifende Gewinde der Gewindehülse als erstes Gewinde bezeichnet werden. Vorzugsweise weisen das erste Gewinde und das zweite Gewinde die gleiche Gewindesteigung auf.

Das vorzugsweise längliche Vortriebsglied kann während der Dosiseinstellung relativ zu dem Gehäuse oder der Zeigeeinrichtung drehfest sein, wodurch die Gewindehülse an dem Gewinde des Vortriebsglieds entlang schraubbar ist. Zum Beispiel ist die Gewindehülse zumindest während der Dosiseinstellung drehfest mit dem Dosierglied verbunden. Die Gewindehülse kann insbesondere mittelbar mit dem Dosisanzeigeelement drehfest, insbesondere permanent drehfest verbunden sein. Vorteilhaft kann die Gewindehülse mit dem Dosisanzeigeelement während der Dosiseinstellung und der Produktausschüttung drehfest verbunden sein.

Um sicherzustellen, dass die Dosisskala in Bezug auf die Zeigeeinrichtung die Schraubbewegung mit der Steigung der Dosisskala ausführt, ist es vorteilhaft, dass die Summe aus der Gewindesteigung des in das Gewinde des Lagerelements eingreifenden Gewindes des Dosisanzeigeelements und der Gewindesteigung des in das Gewinde des Vortriebsglieds eingreifenden ersten Gewindes der Gewindehülse gleich der Steigung der wendelförmigen Skala des Dosisanzeigeelements ist. Hiermit wird sichergestellt, dass die Gewindehülse und somit auch das Lagerelement um den entsprechend der oben genannten Differenz definierten Weg verschoben werden, wenn das Dosierglied gedreht wird.

Alternativ oder zusätzlich kann die Summe aus der Gewindesteigung des in das Gewinde des Lagerelements eingreifenden Gewindes des Dosisanzeigeelements und der Gewindesteigung des in das gehäusefeste Gewinde eingreifenden Gewindes der Gewindehülse gleich der Steigung der wendelförmigen Skala des Dosisanzeigeelements sein. Hierdurch wird die gleiche Wirkung erzielt.

In bevorzugten Ausführungen kann die Gewindehülse während der Dosiseinstellungen relativ zu dem Vortriebsglied drehbar und während der Produktausschüttung relativ zu dem Vortriebsglied drehfest sein. Das Dosisanzeigeelement kann in bevorzugten Ausführungen drehfest und axial verschiebbar mit einem Kupplungsglied, welches während der Dosiseinstellung und vorzugsweise während der Produktausschüttung relativ zu der Zeigeeinrichtung drehbar ist, verbunden sein.

Das Vortriebsglied, das vorzugsweise als Kolbenstange ausgebildet ist, weist ein Außengewinde auf, welches zum Beispiel von einer Längsführung, insbesondere einer Abflachung oder einer Längsnut überlagert sein kann. Zum Beispiel kann das Gewinde des Vortriebsglieds mit einem Gewinde des Gehäuses oder eines gehäusefesten Elements in einem Gewindeeingriff stehen, wodurch eine Drehung des Vortriebsglieds um die Längsachse bewirkt, das das Vortriebsglied entlang der Längsachse bewegt, insbesondere geschraubt wird. Bei der Produktausschüttung wird das Vortriebsglied in eine Drehrichtung gedreht, die bewirkt, dass das Vortriebsglied in das Produktbehältnis hinein verschoben wird und dadurch den Kolben in dem Produktbehältnis verschiebt.

In bevorzugten Ausführungen umfasst die Antriebs- und Dosiervorrichtung eine, insbesondere erste, Kupplung welche geöffnet ist, wenn das Betätigungsglied unbetätigt ist und geschlossen ist, wenn das Betätigungsglied betätigt ist oder welche durch Betätigung des Betätigungsglieds geschlossen wird. Die geschlossene Kupplung verbindet das Kupplungsglied und das Vortriebsglied drehfest. Das Kupplungsglied ist relativ zu dem Vortriebsglied drehbar, wenn die Kupplung geöffnet ist.

Die Kupplung kann eine erste Kupplungsstruktur und eine zweite Kupplungsstruktur umfassen, die, wenn die Kupplung geschlossen ist, ineinander greifen und, wenn die Kupplung geöffnet ist, außer Eingriff sind. Die Kupplung kann beispielsweise eine Klauenkupplung sein. Die erste Kupplungsstruktur und die zweite Kupplungsstruktur können jeweils eine Verzahnung aufweisen, wobei die Verzahnungen zur Bildung einer drehfesten Verbindung ineinander greifen können. Beispielsweise ist die erste Kupplungsstruktur eine Außenverzahnung und die zweite Kupplungsstruktur eine Innenverzahnung, oder umgekehrt. Die erste Kupplungsstruktur kann an dem Kupplungsglied angeordnet sein, wobei die zweite Kupplungsstruktur an einem Rotationsglied gebildet sein kann.

Das Rotationsglied kann zum Beispiel hülsenförmig sein und verdrehfest aber axial verschiebbar mit dem Vortriebsglied verbunden sein. Hierzu kann das Rotationsglied zum Beispiel einen Steg aufweisen, der in die Längsführung, insbesondere die Nut oder die Abflachung, des Vortriebsglieds verdrehfest eingreift. Das Rotationsglied kann zum Beispiel ein federnd angeordnetes Eingriffsglied aufweisen, welches zum Beispiel in der Gestalt eines Nockens an einem Arm ausgebildet sein kann. Das Eingriffsglied des Rotationsglieds kann zum Beispiel in eine Innenverzahnung des Gehäuses oder eines gehäusefesten Elements eingreifen. Das Rotationsglied kann als unidirektionale Kupplung wirken, wobei eine Drehung des Rotationsglieds und somit auch der Kolbenstange nur in die Richtung möglich ist, welche eine Ausschüttbewegung des Vortriebsglieds bewirkt, und in die entgegengesetzte Richtung gesperrt ist. Eine solche unidirektionale Kupplung kann auch als Ratsche bezeichnet werden.

Ist das Betätigungsglied betätigt, bewirkt eine Drehung des Kupplungsglieds, dass das Rotationsglied relativ zu dem Gehäuse oder der Zeigeeinrichtung verdreht wird.

Die Antriebs- und Dosiervorrichtung umfasst alternativ oder zusätzlich eine, insbesondere zweite, Kupplung welche geschlossen ist, wenn das Betätigungsglied unbetätigt ist, und welche geöffnet ist, wenn das Betätigungsglied betätigt ist, oder durch Betätigung des Betätigungsglieds geöffnet wird. Die geschlossene zweite Kupplung kann das Dosierglied und das Kupplungsglied zumindest in eine Drehrichtung drehfest verbinden, vorzugsweise in beide Drehrichtungen, insbesondere abgesehen von gewissen Elastizitäten. Das Kupplungsglied kann bei geöffneter zweiter Kupplung relativ zu dem Dosierglied drehbar sein. Die zweite Kupplung kann eine dritte und vierte Kupplungsstruktur aufweisen, die bei geöffneter zweiter Kupplung außer Eingriff sind und bei geschlossener zweiter Kupplung in einem verdrehgesicherten Eingriff sind. Die zweite Kupplung kann eine Klauenkupplung sein. Die dritte Kupplungsstruktur und die vierte Kupplungsstruktur können jeweils eine Verzahnung aufweisen, wobei die Verzahnungen zur Bildung einer drehfesten Kupplung ineinander greifen können. Beispielsweise ist die dritte Kupplungsstruktur eine Außenverzahnung und die vierte Kupplungsstruktur eine Innenverzahnung, oder umgekehrt. Die dritte Kupplungsstruktur kann zum Beispiel an dem Kupplungsglied und die vierte Kupplungsstruktur kann zum Beispiel an dem Dosierglied oder einem Kupplungselement, welches Teil einer Sperreinrichtung sein kann, gebildet sein.

Besonders vorteilhaft ist es, wenn das Kupplungsglied mittels der ersten Kupplung bereits drehfest mit dem Rotationsglied gekoppelt ist und mittels der zweiten Kupplung noch drehfest mit dem Dosierglied verbunden ist, während das Betätigungsglied für die Betätigung im Bezug auf das Gehäuse verschoben wird. Hierdurch wird sichergestellt, dass das Kupplungsglied sicher mit dem Rotationsglied gekoppelt ist, wenn das Kupplungsglied für eine Drehung relativ zu dem Gehäuse oder dem Dosierglied freigegeben ist. Mit anderen Worten gibt es zwischen der vollständig betätigten Position und der unbetätigten Position des Betätigungsglieds eine Zwischenposition in der das Kupplungsglied sowohl drehfest mit dem Dosierglied als auch drehfest mit dem Rotationsglied gekoppelt ist.

Bevorzugt umfasst die Antriebs- und Dosiervorrichtung eine Kupplungsfeder, welche die erste Kupplung in ihre geöffnete Stellung und/oder die zweite Kupplung in ihre geschlossene Stellung spannt, wenn das Betätigungsglied unbetätigt ist. Bei dieser Kupplungsfeder kann es sich um die erwähnte Kupplungs- oder Rückstellfeder handeln, die durch Betätigung des Betätigungsglieds gespannt wird.

Das insbesondere hülsenförmige Kupplungsglied kann vorzugsweise permanent drehfest, und vorzugsweise axial bewegbar, mit der Gewindehülse und/oder dem Dosisanzeigeelement verbunden sein. Hierdurch wird bewirkt, dass das Dosisanzeigeelement die mittels Drehung des Dosierglieds einstellbare Produktdosis anzeigt und dass das Dosisanzeigeelement bei Betätigung des Betätigungsglieds zum Wert Null herunterzählt.

Die Antriebs- und Dosiervorrichtung umfasst ferner eine Antriebsfeder, welche vorgespannt ist oder mittels Drehung des Dosierglieds vorspannbar ist und das Vortriebsglied während der Produktausschüttung antreibt. Die für das Zurückdrehen des Dosisanzeigeelements und/oder das Bewegen des Vortriebsglieds in distale Richtung benötigte Energie kann automatisch, insbesondere durch die in der Antriebs- und Dosiervorrichtung enthaltenen Feder insbesondere Ausschüttfeder, in der die benötigte Energie gespeichert ist oder werden kann, bereitgestellt werden. Zum Beispiel kann die in der Ausschüttfeder gespeicherte Federenergie bei Betätigung des Betätigungsglieds an das Dosisanzeigeelement und/oder das Vortriebsglied abgegeben werden, sodass das Dosisanzeigeelement zurückgedreht und das Vortriebsglied in distale Richtung bewegt wird. Die Ausschüttfeder ist vorzugsweise so mit dem Dosierglied insbesondere über eine lösbare Kupplung gekoppelt, dass eine Drehung des Dosierglieds bei der Dosiseinstellung, insbesondere bei der Dosiserhöhung oder Drehung in die erste Drehrichtung, die Ausschüttfeder vorspannt. Die Ausschüttfeder kann dann die für die eingestellte Dosis erforderliche Energie speichern.

Eine Drehung des Dosierglieds in eine erste Drehrichtung, die eine Dosisverringerung bewirkt, kann bewirken, dass die Ausschüttfeder entweder entspannt oder nicht entspannt wird. Soll die Ausschüttfeder bei Drehung des Dosierglieds in die erste Drehrichtung entspannt werden, kann ein Ende der Feder in beide Drehrichtungen drehfest, insbesondere über die Kupplung, mit dem Dosierglied gekoppelt sein. Soll die Ausschüttfeder bei Drehung des Dosierglieds in die erste Drehrichtung nicht entspannt werden, ist es vorteilhaft, insbesondere kinematisch zwischen dem Dosierglied und der Feder, insbesondere zusätzlich zu der genannten Kupplung eine unidirektionale Kupplung, insbesondere eine Ratsche anzuordnen, welche die Drehung des Dosierglieds in die zweite Drehrichtung an die Ausschüttfeder überträgt und die Drehung des Dosierglieds in die erste Drehrichtung an die Ausschüttfeder nicht überträgt.

Zum Beispiel kann die Ausschüttfeder eine wendelförmige oder spiralförmige Feder sein, die als Drehfeder wirkt. Die Ausschüttfeder kann sich mit einem Ende an dem Kupplungsglied und an dem mit dem anderen Ende an dem Gehäuse oder einem gehäusefesten Element jeweils drehfest abstützen.

In bevorzugten Ausführungen kann die Antriebs- und Dosiervorrichtung eine lösbare Sperreinrichtung aufweisen, welche so zwischen Dosierglied und Kupplungsglied und insbesondere das Gehäuse geschaltet ist, dass das von der Antriebsfeder auf das Kupplungsglied ausgeübte Drehmoment in das Gehäuse geleitet wird, so dass das Kupplungsglied relativ zu dem Gehäuse verdrehgesichert ist, wobei diese Verdrehsicherung des Kupplungsglieds mittels Drehung des Dosierglieds, insbesondere in die erste Drehrichtung, lösbar ist, sodass das Kupplungsglied relativ zu dem Gehäuse in eine erste und vorzugsweise auch in eine zweite Drehrichtung drehbar ist.

Ferner kann die Antriebs- und Dosiervorrichtung einen Mechanismus zum Verhindern des Einstellens seiner Dosis, welche die Menge eines Medikaments in die Produktbehälter übersteigt, aufweisen. Insbesondere kann dieser Mechanismus die Drehung des Dosierglieds in eine Richtung, welche eine Dosiserhöhung bewirken würde, blockieren, insbesondere auch, wenn der Maximaldosisanschlag des Dosisanzeigeelements der Maximaldosis gegen Anschlag noch nicht in einem Eingriff sind oder wenn in der Zeigeeinrichtung eine Dosis angezeigt wird, die kleiner als die maximal einstellbare Produktdosis ist. Der Mechanismus verhindert somit, dass eine Dosis eingestellt werden kann, welche die ausschüttbare Restmenge des in dem Produktbehälter enthaltenen Produkts übersteigt, wodurch die Gefahr einer Fehlanwendung der Antriebs- und Dosiervorrichtung verringert wird. Der Mechanismus kann zum Beispiel einen Begrenzer aufweisen, der zwischen zwei Teilen angebracht ist, von denen sich eines relativ zu dem anderen während der Dosiseinstellung dreht und bei Betätigung, das heißt der Dosisausschüttung nicht dreht zum Beispiel kann der Begrenzer zwischen dem Dosiseinstellglied, das insbesondere als Dosiseinstellknopf oder Dosiseinstellhülse ausgestaltet sein kann und dem Gehäuse oder einem gehäusefesten Element angeordnet sein. Der Begrenzer, das Dosiseinstellglied und das Gehäuse können so miteinander gekoppelt sein, dass eine relative Drehung, insbesondere während der Dosiseinstellung, zwischen dem Dosiseinstellglied und dem Gehäuse bewirkt, dass sich der Begrenzer zu einer Stoppposition hinbewegt, in welcher der Begrenzer das Einstellen einer Dosis verhindert, die die Menge eines Produkts in dem Produktbehälter übersteigt. Beispiele für entsprechend geeignete Begrenzer werden in der WO 2010/49209 oder in der WO 01/19434 A1, insbesondere in deren Figur 3 offenbart. Beispielsweise kann der Begrenzer ein Innengewinde aufweisen, welches in einem Eingriff mit einem Außengewinde des Gehäuses ist. Insbesondere kann der Begrenzer an seiner Außenseite eine Längsführung aufweisen mit der er in einem Eingriff mit dem Dosiseinstellglied ist, so dass das Dosiseinstellglied relativ zu dem Begrenzer drehfest ist. Alternativ kann das Gehäuse die Längsführung für den Begrenzer aufweisen, so dass der Begrenzer relativ zu dem Gehäuse drehfest ist und kann der Begrenzer ein Gewinde, insbesondere Außengewinde aufweisen, welches in ein Gewinde insbesondere Innengewinde des Dosiseinstellglieds eingreift.

Die Stoppposition wird durch einen Stoppanschlag für den Begrenzer definiert, wobei der Anschlag von dem Gehäuse oder dem Dosiseinstellglied oder einem zumindest axial oder in Umfangsrichtung gehäusefesten Mittel gebildet werden kann. Sind Begrenzer und der Stoppanschlag in einem Kontakt, ist die Drehung des Dosiseinstellglieds die Drehrichtung, welche eine Erhöhung der Dosis bewirken würde, z.B. die zweite Drehrichtung, nicht mehr möglich oder blockiert.

In einer alternativen Ausführung für einen solchen Mechanismus kann der Begrenzer von der Gewindehülse gebildet werden, wobei die Stoppposition von einem Anschlag an dem Vortriebsglied, insbesondere an dem hinteren Ende oder proximalen Ende des Vortriebsglieds definiert wird. Der Anschlag kann ein in Axialrichtung wirkender Anschlag oder ein in Umfangsrichtung wirkender Anschlag sein.

Die Erfindung wurde anhand mehrerer bevorzugter Ausführungen beschrieben. Im Folgenden werden besonders bevorzugte Ausführungen der Erfindung anhand von Figuren beschrieben. Die dabei offenbarten Merkmale bilden je einzeln und in jeglicher Merkmalskombination, insbesondere auch mit den oben beschriebenen Merkmalen, den Gegenstand der Ansprüche vorteilhaft weiter. Es zeigen:
- Figur 1: eine Explosionsdarstellung der Einzelteile einer Injektionsvorrichtung mit einer Antriebs- und Dosiervorrichtung nach einer ersten Ausführungsform,

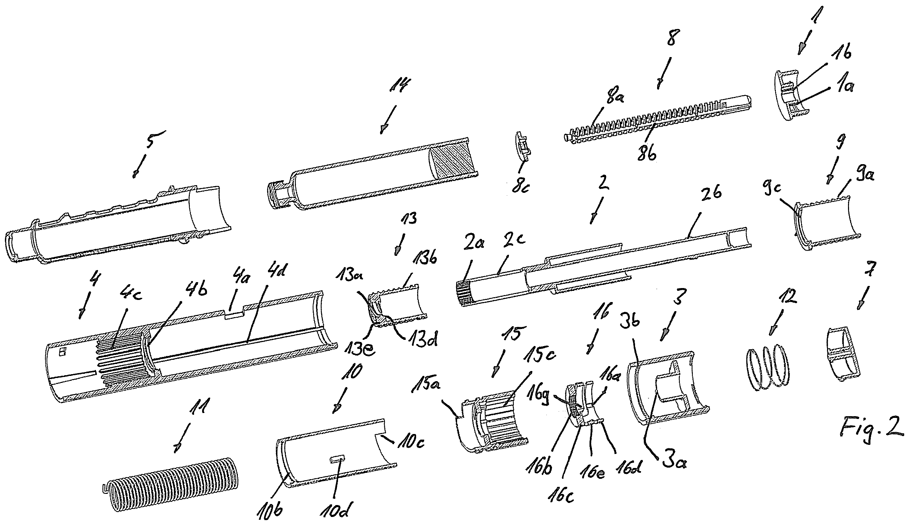

- Figur 2: die Darstellung aus Figur 1, wobei die Einzelteile im Schnitt dargestellt sind,
- Figuren 3a bis 3d: verschiedene Ansichten einer aus den Einzelteilen aus den Figuren 1 und 2 zusammengesetzten Injektionsvorrichtung in einem Ausgangs-oder Auslieferungszustand,
- Figuren 4a bis 4d: die Ansichten der Vorrichtung aus den Figuren 3a bis 3d mit einer maximal eingestellten Dosis,
- Figuren 5 bis 5d: die Ansichten der Vorrichtung aus den Figuren 3a bis 3d nach Ausschüttung der eingestellten Produktdosis,
- Figuren 6a bis 6d: die Ansichten der Vorrichtung aus den Figuren 3a bis 3d in einem Zustand, bei dem die in dem Produktbehälter enthaltene ausschüttbare Produktdosis geringer als die mit der Vorrichtung maximal einstellbare Dosis ist,
- Figur 7: eine Explosionsdarstellung der Einzelteile einer Injektionsvorrichtung mit einer Antriebs- und Dosiervorrichtung nach einer zweiten Ausführungsform,
- Figur 8: die Darstellung aus Figur 7, wobei die Einzelteile im Schnitt dargestellt sind,
- Figuren 9a bis 9d: verschiedene Ansichten einer aus den Einzelteilen aus den Figuren 7 und 8 zusammengesetzten Injektionsvorrichtung in einem Ausgangs-oder Auslieferungszustand,
- Figuren 10a bis 10d: die Ansichten der Vorrichtung aus den Figuren 9a bis 9d mit einer maximal eingestellten Dosis,
- Figuren 11a bis 11d: die Ansichten der Vorrichtung aus den Figuren 9a bis 9d nach Ausschüttung der eingestellten Produktdosis,
- Figuren 12a bis 12d: die Ansichten der Vorrichtung aus den Figuren 9a bis 9d in einem Zustand, bei dem die in dem Produktbehälter enthaltene ausschüttbare Produktdosis geringer als die mit der Vorrichtung maximal einstellbare Dosis ist,
- Figur 13: eine Explosionsdarstellung der Einzelteile einer Injektionsvorrichtung mit einer Antriebs- und Dosiervorrichtung nach einer dritten Ausführungsform,
- Figur 14: die Darstellung aus Figur 13, wobei die Einzelteile im Schnitt dargestellt sind,
- Figuren 15a bis 15d: verschiedene Ansichten einer aus den Einzelteilen aus den Figuren 13 und 14 zusammengesetzten Injektionsvorrichtung in einem Ausgangs-oder Auslieferungszustand,
- Figuren 16a bis 16d: die Ansichten der Vorrichtung aus den Figuren 16a bis 16d mit einer maximal eingestellten Dosis,
- Figuren 17a bis 17d: die Ansichten der Vorrichtung aus den Figuren 16a bis 16d nach Ausschüttung der eingestellten Produktdosis,
- Figuren 18a bis 18d: die Ansichten der Vorrichtung aus den Figuren 16a bis 16d in einem Zustand, bei dem die in dem Produktbehälter enthaltene ausschüttbare Produktdosis geringer als die mit der Vorrichtung maximal einstellbare Dosis ist.

Zunächst werden die gemeinsamen Merkmale der ersten, zweiten und dritten Ausführungsform beschrieben. Anschließend werden die speziellen Merkmale der jeweiligen Ausführungsform beschrieben.

Die in den Figuren gezeigten Ausführungsformen weisen ein hülsenförmiges Gehäuse 4 auf, in dem ein fensterartiger Durchbruch zur Bildung einer Zeigeeinrichtung 4a angeordnet ist. An dem distalen, das heißt vorderen Ende des Gehäuses 4 ist ein Produktbehälterhalter 5 formschlüssig, vorzugsweise unlösbar befestigt, insbesondere verschnappt, welcher einen Produktbehälter 14 in der Gestalt einer Karpule aufnimmt. Die Karpule weist ein zylindrisches Gehäuse auf, in dem ein Kolben veschiebbar angeordnet ist. Am distalen Ende weist die Karpule ein mit einer Nadel durchstechbares Septum auf. Zwischen dem Septum und dem Kolben befindet sich das zu verabreichende Produkt. Durch Verschiebung des Kolbens Richtung Septum wird das Produkt aus dem Produktbehälter 14 verdrängt. An dem proximalen Ende des Produktbehälterhalters 5 ist ein Gewinde oder ein Bajonettverschluss gebildet, an dem die Nadel befestigbar ist. Auf den Produktbehälterhalter 5 kann eine Kappe 6 abnehmbar aufgesteckt werden.

An dem proximalen, das heißt hinteren Ende des Gehäuses 4 ist ein relativ zu dem Gehäuse 4 drehbares Dosierglied 3 angeordnet, welches eine äußere Oberfläche der Vorrichtung bildet und vom Verwender der Vorrichtung ergreifbar ist und relativ zu dem Gehäuse 4 verdrehbar ist. Eine Drehung des Dosierglieds 3 in eine erste Drehrichtung bewirkt eine Verringerung der Dosis, wobei die Drehung des Dosierglieds in eine zweite Drehrichtung eine Erhöhung der Dosis bewirkt. Das Dosierglied 3 ist axialfest mit dem Gehäuse 4 verbunden. An dem Gehäuse 4 ist ein Gehäuseeinsatz 15 dreh und axialfest befestigt, der somit gehäusefest ist und dem Gehäuse 4 zugerechnet werden kann. Der Gehäuseeinsatz 15 weist eine Ringnut 15b auf, in die ein Ringsteg 3b am inneren Umfang des Dosierglieds 3 einrastet, wodurch das Dosierglied 3 drehbar und axialfest mit dem Gehäuseeinsatz 15 verbunden ist. Der Gehäuseeinsatz 15 ist hülsenförmig.

Insbesondere am distalen Ende des Gehäuseeinsatzes 15 ist ein in Umfangsrichtung wirkender Maximaldosisgegenanschlag 15a für einen Maximaldosisanschlag 10c eines Dosisanzeigeelements 10 gebildet.

Über den inneren Umfang weist der hülsenförmige Gehäuseeinsatz 15 eine Verzahnung 15c auf. Der Gehäuseeinsatz 15 umgibt ein einstückiges Kupplungselement 16, das vorzugsweise aus Kunststoff hergestellt und/oder ein Spritzgussteil ist. Das Kupplungselement 16 weist einen ersten Abschnitt 16c und einen zweiten Abschnitt 16d auf. Der erste Abschnitt 16c ist mit dem zweiten Abschnitt 16d über einen elastisch verformbaren Zwischenabschnitt 16e verbunden. Der Zwischenabschnitt 16e mündet mit einem Ende in den ersten Abschnitt 16c und mit dem anderen Ende in den zweiten Abschnitt 16d. Der Zwischenabschnitt 16e erstreckt sich von dem ersten Abschnitt 16c in die erste Drehrichtung und von dem zweiten Abschnitt 16d entgegen der ersten Drehrichtung, das heißt in die zweite Drehrichtung. Der Zwischenabschnitt 16e ist vorzugsweise länglich und erstreckt sich zumindest über einen Teil des Umfangs des Kupplungselements 16. Das Kupplungselement 16 weist zwei solche Zwischenabschnitte 16e auf, die insbesondere um 180° versetzt über den Umfang angeordnet sind.

Der oder die Zwischenabschnitte 16e weisen, insbesondere jeweils ein Eingriffsglied 16f auf, welches in die Verzahnung 15c der Hülse 15 eingreift. Das Eingriffsglied 16f ist ein insbesondere radial vom Umfang des Kupplungselements 16 nach außen ragender Zahn oder Nocken gebildet.

Das Eingriffsglied 16f weist eine in die erste Drehrichtung weisende, erste Zahnflanke und eine in die zweite Drehrichtung weisende, zweite Zahnflanke auf. Die erste und zweite Zahnflanke sind unterschiedlich steil angeordnet, so dass das Eingriffsglied 16f sägezahnförmig ist. Die erste Flanke ist vorzugsweise steiler als die zweite Flanke angeordnet.

Das Eingriffsglied 16f ist vorzugsweise zwischen den Enden des Zwischenabschnitts 16e, vorzugsweise mittig, angeordnet.

Die Verzahnung 15c weist eine Vielzahl über den im Umfang verteilte Zähne auf. Ein Zahn oder mehrere, insbesondere jeder dieser Zähne können zum Beispiel sägezahnförmig gebildet sein. Sie können eine in Umfangsrichtung weisende erste Flanke und eine entgegengesetzt zu der ersten Flanke in Umfangsrichtung weisende zweite Flanke aufweisen, wobei die erste Flanke steiler als die zweite Flanke ausgebildet ist. Vorzugsweise bildet die erste Flanke eine Gegenflanke für die erste Flanke des Eingriffsglieds 16f.

Ein Drehmomentbeaufschlagungsmittel, in diesem Beispiel eine als Wendelfeder ausgestaltete, als Drehfeder wirkende und als Antriebsfeder dienende Feder 11, stützt sich mit einem, insbesondere dem distalen Ende, an einem relativ zu dem Gehäuse 4 drehbaren Kupplungsglied 2 und mit dem anderen, insbesondere dem proximalen Ende gehäusefest, insbesondere an dem Gehäuseeinsatz 15 ab. Eine Drehung des Kupplungsglieds 2 in die zweite Drehrichtung bewirkt ein Spannen der Feder 11, wobei eine Drehung in die erste Drehrichtung ein Entspannen der Feder 11 bewirkt. Das Kupplungsglied 2 ist vorzugsweise über eine lösbare zweite Kupplung 2b, 16b im Wesentlichen verdrehgesichert mit dem Dosierglied 3 verbunden. Das Kupplungsglied 2 weist zur Bildung der zweiten Kupplung 2b, 16b eine Kupplungsstruktur 2b auf. Der erste Abschnitt 16c des Kupplungselements 16 weist zur Bildung der zweiten Kupplung 2b, 16b eine Kupplungsstruktur 16b auf, welche bei geschlossener zweiter Kupplung 2b, 16b in einem verdrehgesicherten Eingriff mit der Kupplungsstruktur 2b ist.

Das Dosierglied 3 kann in einer ersten Variante permanent oder in beide Drehrichtungen drehfest mit dem zweiten Abschnitt 16d des Kupplungselements 16 verbunden sein. Für die verdrehgesicherte Verbindung weist der zweite Abschnitt 16d an seinem Innenumfang eine Nut 16a auf, in welche eine Abragung 3a des Dosierglieds 3 eingreift. Optional kann an dem ersten Abschnitt 16c oder dem Zwischenabschnitt 16e, insbesondere zwischen Eingriffsglied 16f und erstem Abschnitt 16c oder dort, wo der Zwischenabschnitt 16e in den ersten Abschnitt 16c mündet, ein Anschlag 16g gebildet sein, gegen den ein Gegenanschlag des Dosierglieds 3, der von der rippenförmigen Abragung 3a gebildet sein kann, gedrückt wird, wenn das Dosierglied 3 in die zweite Drehrichtung gedreht wird. Hierdurch wird das auf das Dosierglied 3 wirkende Drehmoment an den ersten Abschnitt 16c übertragen, ohne über den gesamten Zwischenabschnitt 16e laufen zu müssen. Hierdurch kann das Kupplungselement 16 noch einfacher relativ zu der Verzahnung 15c in die zweite Drehrichtung verdreht werden.

In einer zweiten Variante kann das Dosierglied 3 in die erste Drehrichtung drehfest mit dem zweiten Abschnitt 16d des Kupplungselements 16 und in die zweite Drehrichtung drehfest mit dem ersten Abschnitt 16c oder dem Verbindungsabschnitt 16e verbunden sein. Z.B. kann das Dosierglied 3 somit, insbesondere in einem beschränkten Maß, wie z.B. wenige Winkelgrade, relativ zu dem zweiten Abschnitt 16d verdrehbar sein. Für diese Verbindung weist der zweite Abschnitt 16d an seinem Innenumfang einen Anschlag 16a auf, an welchen ein Gegenanschlag, insbesondere eine Abragung oder der Längssteg 3a des Dosierglieds 3, in die erste Drehrichtung anschlägt. Weiter weist der Zwischenabschnitt 16e im Bereich der Verbindungsstelle zum ersten Abschnitt 16c einen Anschlag 16g auf, an welchen ein Gegenanschlag, insbesondere die Abragung oder der Längssteg 3a, in die zweite Drehrichtung anschlägt.

Durch die zwei Varianten wird bewirkt, dass das durch das Dosierglied 3 auf das Kupplungselement 16 ausgeübte Drehmoment über den gesamten Verbindungsabschnitt 16e läuft, wenn das Dosierglied 3 in die erste Drehrichtung gedreht wird, so dass sich der Verbindungsabschnitt 16e elastisch verformen kann. Ferner wird bewirkt, dass das durch das Dosierglied 3 auf das Kupplungselement 16 ausgeübte Drehmoment an dem oder in den ersten Abschnitt 16d, ohne über den gesamten Verbindungsabschnitt 16e laufen zu müssen, eingeleitet wird, wenn das Dosierglied 3 in die erste Drehrichtung gedreht wird.

Allgemein gilt, dass das Drehmomentbeaufschlagungsmittel, insbesondere die Feder 11, den ersten Abschnitt 16c des Kupplungselements 16 um die Längsachse L mit einem in die erste Drehrichtung gerichteten Drehmoment beaufschlagt. Die Höhe des Drehmoments hängt davon ab, wie stark die Feder 11, insbesondere die Drehfeder, rotatorisch vorgespannt ist. Das Eingriffsglied 16f wird durch das in die erste Drehrichtung wirkende Drehmoment in die Verzahnung 15c gedrückt, so dass eine Drehnung des Kupplungselements 16 relativ zu dem Gehäuseeinsatz 15 in die erste Drehrichtung blockiert wird. Damit das Eingriffsglied 16f sicher in die Verzahnung 15c gedrückt wird, ist es besonders vorteilhaft, dass sich der Zwischenabschnitt 16e von dem ersten Abschnitt 16c in Umfangsrichtung in die erste Drehrichtung erstreckt. Als alternative oder zusätzliche Maßnahme können die erste Flanke des Eingriffsglieds 16f und die erste Flanke eines der Zähne der Verzahnung 15c so aufeinander abgestimmt werden, dass zwischen dem Eingriffsglied 16f und der Verzahnung 15c eine Selbsthemmung auftrifft, das heißt dass auch bei einem sehr hohen Drehmoment das Eingriffsglied 16f nicht aus der Verzahnung 15c gedrückt wird. Die Flanken können auch so aufeinander abgestimmt sein, dass das Eingriffsglied 16f in den Eingriff mit der Verzahnung 15c hineingezogen wird.

Der zweite Abschnitt 16d des Kupplungselements 16 ist relativ zu dem ersten Abschnitt 16c um die Längsachse L in die erste Drehrichtung insbesondere elastisch tordierbar, insbesondere mittels Drehung des Dosierglieds 3 in die erste Drehrichtung. Die Tordierbarkeit wird durch den elastischen Zwischenabschnitt 16e bereitgestellt. Durch die Torsion des zweiten Abschnitts 16d relativ zu dem ersten Abschnitt 16c wird der die Verdrehung in die erste Drehrichtung sichernde Eingriff des Eingriffsglieds 16f in die Verzahnung 15c gelöst. Durch die Torsion wird das Eingriffsglied 16f ein kleines Stück nach innen, das heißt zur Drehachse L hin ausgelenkt, so dass das Eingriffsglied 16f aus dem verdrehgesicherten Eingriff gelöst wird. Somit ist eine Drehung des Kupplungselements 16 relativ zu der Hülse 15 in die erste Drehrichtung freigegeben. Das Eingriffsglied 16f wird somit den Zahn der Verzahnung 15c, mit dem es im Eingriff war, überspringen und in den nächsten, insbesondere nächstbenachbarten Zahn eingreifen. Somit lässt sich das Kupplungselement 16 relativ zu dem Gehäuseeinsatz 15 inkrementweise oder um ein Winkelinkrement entsprechend der Zahnteilung der Verzahnung 15c in die erste Drehrichtung drehen.

Hierdurch lässt sich vorteilhaft mit einem einfach gestalteten Bauteil, nämlich dem hülsenförmigen Kupplungselement 16 das Aufdosieren und das Abdosieren ermöglichen sowie das Entspannen der Feder 11 verhindern.

Durch Drehen des Dosierglieds 3 in die zweite Drehrichtung rastet das Eingriffsglied 16f über die Zähne der Verzahnung 15c. Durch die geschlossene Kupplung 2b, 16b wird die Feder 11 gespannt.

Um die gewünschte Elastizität des Zwischenabschnitts 16e zu erhalten, kann der Zwischenabschnitt 16e eine andere Wandstärke als der erste und/oder zweite Abschnitt 16c, 16d aufweisen. In dem gezeigten Beispiel ist die Wandstärke des Zwischenabschnitts 16e geringer als die Wandstärke des ersten Abschnitts 16c.

Am proximalen Ende der Antriebs- und Dosiervorrichtung ist ein als Betätigungsknopf ausgestaltetes Betätigungsglied 7 angeordnet, welches durch den Verwender der Vorrichtung für eine Produktausschüttung betätigbar, insbesondere in distale Richtung drückbar ist. Das Betätigungsglied 7 ist im Bezug auf das Dosierglied 3 so angeordnet, dass es seine Axialposition während der Dosiseinstellung hierzu nicht ändert. Das Betätigungsglied 7 ist insbesondere um einen Betätigungshub verschiebbar in dem Dosierglied 3 aufgenommen. Zwischen dem Dosierglied 3 und dem Betätigungsglied 7 wirkt eine Kupplungs- oder Rücksetzfeder 12, welche als Wendelfeder ausgestaltet ist und als Druckfeder wirkt. Die Feder 12 stützt sich an dem Dosierglied 3 und dem Betätigungsglied 7 ab. Das Betätigungsglied 7 bildet das proximale Ende der Antriebs- und Dosiervorrichtung.

Das Betätigungsglied 7 ist zumindest axialfest vorzugsweise auch drehfest mit dem Dosierglied 3 und mit dem proximalen Ende des Kupplungsglieds 2 axialfest verbunden, insbesondere verschnappt. Das Betätigungsglied 7 ist zwischen einer betätigten und einer unbetätigten Position hin und her verschiebbar.

Die Antriebs- und Dosiervorrichtung weist ein Vortriebsglied 8 in der Gestalt einer Kolbenstange auf, an deren distalem Ende ein Flansch 8c angeordnet bzw. befestigt ist. Das Vortriebsglied 8 wirkt auf den Kolben des Produktbehälters 14 oder stößt bevorzugt an dem Kolben an. Das Vortriebsglied 8 weist ein Außengewinde 8a auf, welches von einer Nut 8b überlagert ist, die sich in Längsrichtung des länglichen Vortriebsglieds 8 erstreckt.

Das Vortriebsglied 8 wird von einem Rotationsglied 1 umgeben, welches vorzugsweise hülsenförmig ist und drehfest und axial verschiebbar in Bezug auf das Vortriebsglied 8 ist. Das Rotationsglied 1 weist einen Steg 1b auf, welcher für die rotationsfeste und axial verschiebbare Verbindung in die Nut 8b eingreift. Das Rotationsglied 1 weist an seinem Umfang ein nockenförmiges, an einem Federarm federnd angeordnetes Eingriffsglied 1c auf, welches in einem Eingriff mit einer gehäusefesten, insbesondere von dem Gehäuse 4 gebildeten Innenverzahnung 4c ist.

Das Gewinde des Vortriebsglieds 8 ist in einem Gewindeeingriff mit einem Innengewinde 13a einer Gewindehülse 13, so dass die Gewindehülse 13 an dem Vortriebsglied 8 entlang schraubbar ist. Die Gewindehülse 13 ist drehfest und axial verschiebbar mit dem Kupplungsglied 2 verbunden, so dass das Kupplungsglied 2 die Gewindehülse 13 mitdreht. Hierzu kann in der Gewindehülse 13 eine Innenhülse 13d gebildet sein, welche über mindestens einen, vorzugsweise zwei oder mehrere Stege 13e speichenartig mit dem Innenumfang der Gewindehülse 13 verbunden ist. Der mindestens eine Steg 13e greift durch je einen, vorzugsweise zwei oder mehrere axial sich erstreckende Führungsschlitze 2c der Kupplungshülse 2 und realisieren so die drehfeste Verbindung mit dieser und eine axiale Beweglichkeit, vorzugsweise für den Betätigungs- oder Kupplungshub, derselben.

Die Gewindehülse 13 ist an einem Lagerelement 9 drehbar und axialfest befestigt. Hierfür kann die Gewindehülse 13 zum Beispiel eine Ringnut 13c aufweisen, in welche das Lagerelement 9 zum Beispiel mit einem Ringsteg 9c eingreift. Das Lagerelement 9 ist im Bezug auf das Gehäuse 4 verdrehgesichert und axial verschiebbar. Das Lagerelement 9 greift für die verdrehfeste und verschiebbare Verbindung mit dem Gehäuse 4 insbesondere mit einer Abragung 9b in eine Nut 4d, die sich entlang der Längsachse L erstreckt.

Das Lagerelement 9 weist ein Außengewinde 9a auf, welches in einem Gewindeeingriff mit einem Innengewinde 10b als Dosisanzeigetrommel gebildeten Dosisanzeigeelements 10 steht. Somit ist das Dosisanzeigeelement 10 an dem Lagerelement 9 entlang schraubbar.

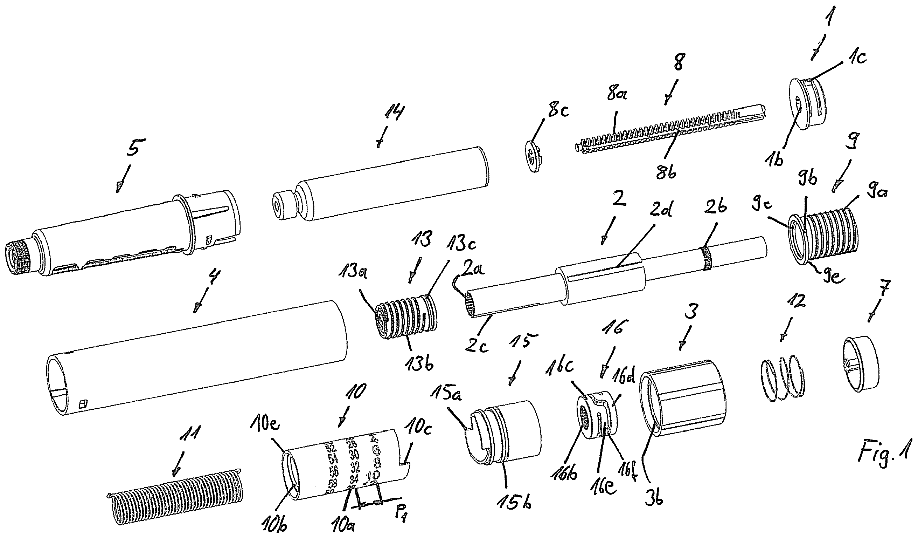

Das Dosisanzeigeelement 10 weist über seinen Umfang eine mehrfach umlaufende, wendelförmige oder helixförmige Dosisskala 10a auf, welche aus einer Vielzahl aneinander gereihten, insbesondere in internationalen Einheiten (IU) angegebenen Dosiswerten gebildet wird. Wie zum Beispiel aus den Figuren 1, 7 und 13 erkennbar ist, kann die Dosisskala 10a zum Beispiel in Einser- oder Zweierschritten die einstellbaren Dosiswerte 0 bis 60 oder 80 IU aufweisen. Durch Drehung des Dosierglieds 3 relativ zu dem Gehäuse 4 oder der Zeigeeinrichtung 4a kann die gewünschte auszuschüttende Produktdosis eingestellt werden, wobei der entsprechende Dosiswert an der Zeigeeinrichtung 4a ablesbar ist oder in der Zeigeeinrichtung 4a erscheint.

Das Dosisanzeigelement 10 ist insbesondere permanent drehfest und axial verschiebbar in Bezug auf das Kupplungsglied 2.

Das Kupplungsglied 2 ist insbesondere mit einer ersten Kupplung 1a, 2a mit dem Rotationsglied 1 verdrehgesichert verbindbar. Hierzu weist das Kupplungsglied 2 an seinem distalen Ende eine Kupplungsstruktur 2a in der Gestalt einer Innenverzahnung auf. Das Rotationsglied 1 weist eine Kupplungsstruktur 1a in der Gestalt einer Außenverzahnung auf. Bei unbetätigtem Betätigungsglied 7 ist die erste Kupplung 1a, 2a geöffnet und die zweite Kupplung 2b, 16b geschlossen, so dass das Kupplungsglied 2 relativ zu dem Rotationsglied 1 und/oder zu dem Vortriebsglied 8 drehbar ist, wobei das Dosierglied 3 im Wesentlichen - abgesehen von einer gewissen Elastizität des Kupplungselements 16 - verdrehgesichert mit dem Kupplungsglied 2 verbunden ist. Ist das Betätigungsglied 7 insbesondere vollständig betätigt, ist die erste Kupplung 1a, 2a geschlossen, wodurch das Kupplungsglied 2 relativ zu dem Rotationsglied 1 und/oder zu dem Vortriebsglied 8 verdrehgesichert ist, und die zweite Kupplung 2b, 16b geöffnet, wodurch das Kupplungsglied 2 relativ zu dem Dosierglied 3 und/oder zu dem Gehäuse 4 drehbar ist. Zwischen der unbetätigten und der vollständig betätigten Position des Betätigungsglieds 7 gibt es eine Zwischenposition, bei der die erste Kupplung 1a, 2a und die zweite Kupplung 2b, 16b geschlossen sind. Hierdurch wird vorteilhaft verhindert, dass das Kupplungsglied 2 für eine Drehung relativ zu dem Gehäuse 4 bereits freigegeben ist, wenn die erste Kupplung 1a, 2a noch nicht geschlossen ist. Dies würde nämlich zu einer Fehlfunktion der Antriebs- und Dosiervorrichtung führen.

Sobald die zweite Kupplung 2b, 16b geöffnet ist, kann die vorgespannte Feder 11 das Kupplungsglied 2 und über die geschlossene erste Kupplung 1a, 2a das Rotationsglied 1 und das Vortriebsglied 8 relativ zu dem Gehäuse 4 verdrehen, wodurch das Vortriebsglied 8 in Ausschüttrichtung, das heißt in Richtung Kolben verschoben wird und die eingestellte Dosis ausschüttet.

Für die Dosiseinstellung, das heißt bei unbetätigtem Betätigungsglied 7 ist das Kupplungsglied 2 rotatorisch von dem Vortriebsglied 8 entkoppelt, sodass Dosierbewegungen keine Ausschüttbewegung des Vortriebsglieds 8 bewirken. Die Steigung P₁ der Dosisskala 10a ist größer als die Steigung der Gewinde 10b, 9a. Damit sich das Dosisanzeigeelement 10 entsprechend der Steigung P₁ der Dosisskala 10a auf einer wendel- oder helixförmigen Bahn bewegt, welche die gleiche Steigung wie die Dosisskala 10 aufweist, wird das Lagerelement 9 von der Gewindehülse 13 um die Differenz zwischen der Steigung P₁ und der Steigung des Gewindes 10b verschoben. Hierfür kann die Gewindehülse 13 ein Gewinde 13a, 13b aufweisen, welches eine Steigung aufweist, die gleich der Differenz aus der Steigung P₁ und der Steigung des Gewindes 10b, 9a ist. Mit anderen Worten ergibt die Summe aus dem Gewinde 13a, 13b und dem Gewinde 10b, 9a die Steigung P₁ der Dosisskala 10a.

Die Gewindehülse 13 ist während der Dosiseinstellung relativ zu dem Vortriebsglied 8 drehbar und während der Dosisausschüttung relativ zu dem Vortriebsglied 8 nicht drehbar.

Bei der Antriebs- und Dosiervorrichtung nach der ersten Ausführungsform weist die Gewindehülse 13 zusätzlich zu dem Innengewinde 13a, in welches das Außengewinde 8a des Vortriebsglieds 8 eingreift, ein Außengewinde 13b mit gleicher Gewindesteigung wie das Gewinde 13a auf. Das Außengewinde 13b greift in ein gehäusefestes Gewinde 4b, welches von dem Gehäuse 4 gebildet wird, ein. Hierdurch wird bewirkt, dass sich die Gewindehülse 13 während der Dosiseinstellung relativ zu dem Gehäuse 4 um den gleichen Weg entlang der Längsachse L bewegt, wie es sich relativ zu dem Vortriebsglied 8 bewegt. Durch die nach der ersten Ausführungsform ausgestaltete Gewindehülse 13 braucht das Vortriebsglied 8 in keinem unmittelbaren Gewindeeingriff mit dem Gehäuse 4 zu sein.

Das Kupplungsglied 2 weist eine sich in Längsrichtung erstreckende Nut 2d auf, in welche eine nach innen ragende Abragung 10d des Dosisanzeigeelements 10 eingreift, wodurch das Dosisanzeigeelement 10 relativ zu dem Kupplungsglied 2 drehfest aber axial verschiebbar ist.

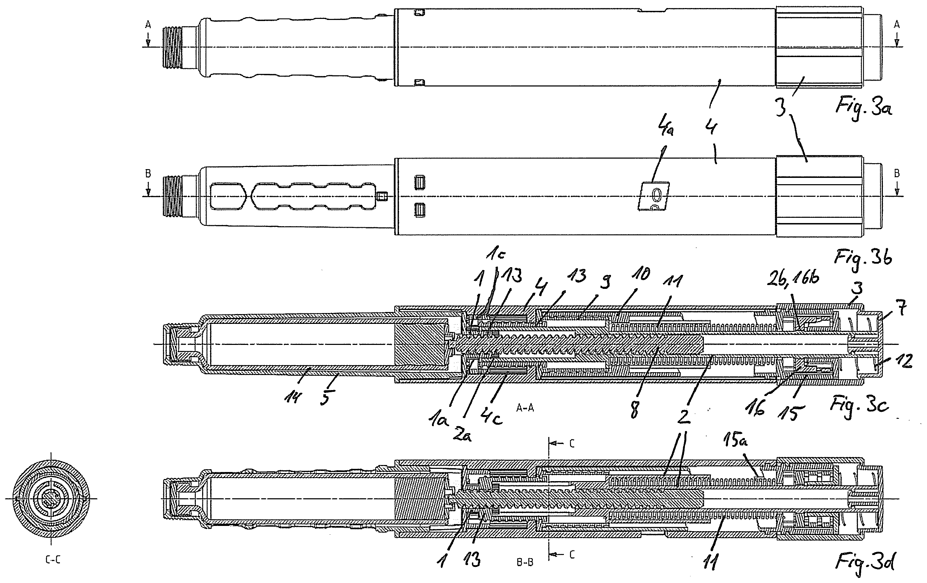

Bezugnehmend auf die Figuren 3a-d wird die Antriebs- und Dosiervorrichtung, die zusammen mit dem Produktbehälter 14 und dem Produktbehälterhalter 5 eine Injektionsvorrichtung bildet, in einer Ausgangsposition oder in einem Auslieferungszustand gezeigt, wobei die Dosis Null eingestellt ist. Das Betätigungsglied 7 ist unbetätigt, wobei die Feder 12 das Betätigungsglied 7 in proximale Richtung drückt, so dass die zweite Kupplung 2b, 16b geschlossen und die erste Kupplung 1a, 2a geöffnet ist. Die als Ausschüttfeder wirkende Feder 11 ist vorzugsweise im Auslieferungszustand etwas vorgespannt.

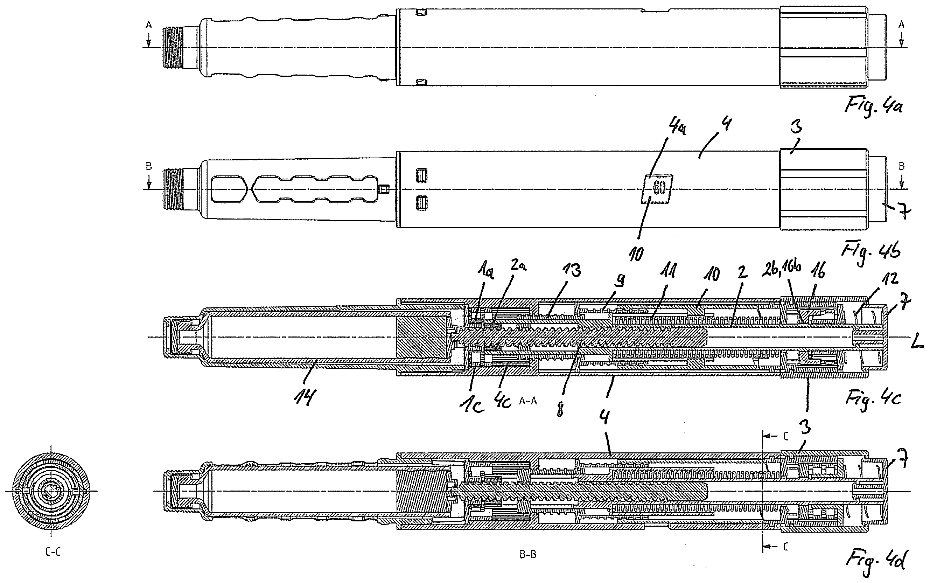

Durch Drehung des Dosierglieds 3 in die zweite Drehrichtung wird über die geschlossene zweite Kupplung 2b, 16b das Kupplungsglied 2 relativ zu dem Gehäuse 4 gedreht, wodurch sich die Gewindehülse 13 mit ihrem Außengewinde 13b an dem Gehäuse 4 und mit ihrem Innengewinde 13a an dem Vortriebsglied 8 entlangschraubt, insbesondere in proximale Richtung, wodurch es das Lagerelement 9 in Axialrichtung mitnimmt und sich das Lagerelement 9 verdrehgesichert entlang des Gehäuses 4 in proximale Richtung verschiebt. Durch die Drehung des Kupplungsglieds 2 wird das Dosisanzeigeelement 10 mitgedreht und schraubt sich somit mit seinem Gewinde 10b an dem Lagerelement 9 entlang. Der Schraubbewegung des Dosisanzeigeelements 10 relativ zu dem Lagerelement 9 wird die Axialbewegung des Lagerelements 9 relativ zu dem Gehäuse 4 überlagert, wodurch das Dosisanzeigeelement 10 in Bezug auf das Gehäuse 4 eine wendel- oder helixförmige Bahn beschriebt, die der Steigung P1 der Dosisskala 10a entspricht. Bei der Dosiserhöhung wird der Maximaldosisanschlag 10c zu dem Maximaldosisgegenanschlag 15a hinbewegt. Ist die mit der Antriebs- und Dosiervorrichtung maximal einstellbare Dosis, wie hier beispielsweise mit 60 IU angegeben, erreicht, schlägt der Maximaldosisanschlag 10c an den Maximaldosisgegenanschlag 15a an (Figuren 4a-d). Durch Drehung des Dosierglieds 3 in die erste Drehrichtung kann die eingestellte Dosis korrigiert oder verringert werden, wobei sich der Maximaldosisanschlag 10c von dem Maximaldosisgegenanschlag 15a wegbewegt und sich ein Nulldosisanschlag 10e, der durch die Stirnseite des Dosisanzeigeelements 10 gebildet wird und als Axialanschlag wirkt, zu einem Nulldosisgegenanschlag 9e, der von einem Kragen des Lagerelements 9 gebildet wird, hinbewegen.

Bei der Drehung des Dosierglieds 3 in die erste Drehrichtung wird die Feder 11 entspannt, wobei sie bei der Drehung in die zweite Drehrichtung gespannt wird. Lässt der Verwender das Dosierglied 3 los, verhindert das Kupplungselement 16, dass sich die Feder 11 entspannt.

Zum Ausschütten der eingestellten Dosis wird das Betätigungsglied 7 gegen die Kraft der Feder 12 in distale Richtung verschoben, wodurch die erste Kupplung 1a, 2a geschlossen und die zweite Kupplung 2b, 16b geöffnet wird. Die Feder 11 treibt nun das Kupplungsglied 2 in die erste Drehrichtung rotatorisch an, wobei das Vortriebsglied 8 relativ zu der Gewindehülse 13 still steht, wobei sich das Vortriebsglied 8 zusammen mit der Gewindehülse 13 mittels des Gewindes 13b an dem Gehäuse 4 in distale Richtung schraubt und somit den Kolben in den Produktbehälter 14 verschiebt. Hierbei wird das Eingriffsglied 1c über die Verzahnung 4c bewegt, wodurch die Ausschüttbewegung mittels Klickgeräuschen signalisiert wird. Das Eingriffsglied 1c bildet mit der Verzahnung 4c ferner eine unidirektionale Kupplung, die bewirkt, dass das Rotationsglied 1 nur in eine Richtung, nämlich in die erste Drehrichtung drehbar ist, welche eine Produktausschüttung bewirkt.

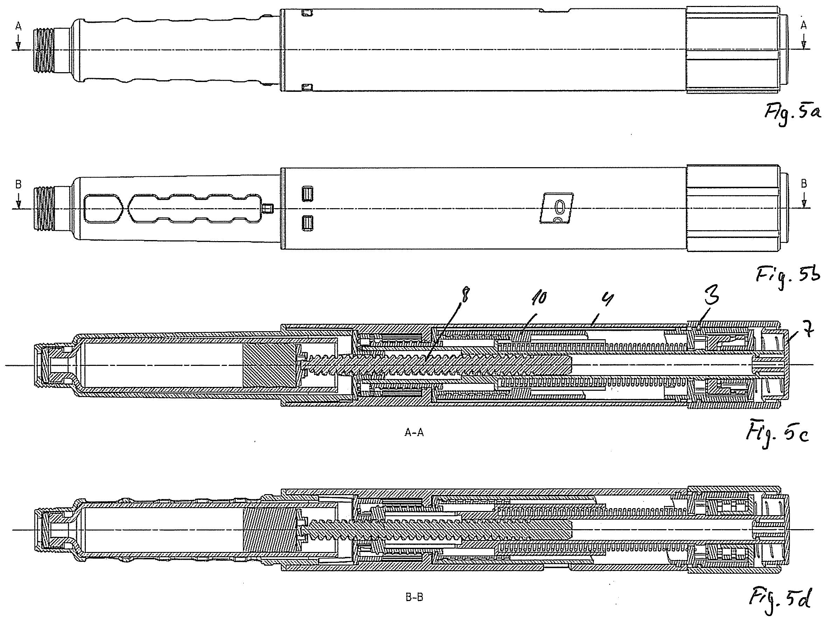

Durch die Drehbewegung des Kupplungsglieds 2 in die erste Drehrichtung wird das Dosisanzeigeelement 10 an dem Lagerelement 9 zurückgeschraubt, insbesondere der Nulldosisanschlag 10e in Richtung Nulldosisgegenanschlag 9e bewegt, wodurch die Dosisskala 10a in dem Zeigeelement 4 zur Nulldosis hin zurück zählt. Wenn die Dosis Null in der Zeigeeinrichtung 4a angezeigt wird (Figuren 5a-d) oder der Nulldosisanschlag 10e an dem Nulldosisgegenanschlag 9e anschlägt, ist die Ausschüttung beendet. In den Figuren 5a, 5b wird die Antriebs- und Dosiervorrichtung am Ende der Produktausschüttung gezeigt, wobei das Betätigungsglied 7 noch betätigt ist, d. h. vom Verwender der Vorrichtung noch nicht losgelassen wurde.

Durch wiederholtes Dosieren und Betätigen des Betätigungsglieds kann das in dem Produktbehälter 14 enthaltene Produkt in mehreren beliebig wählbaren Einzeldosen ausgeschüttet werden.

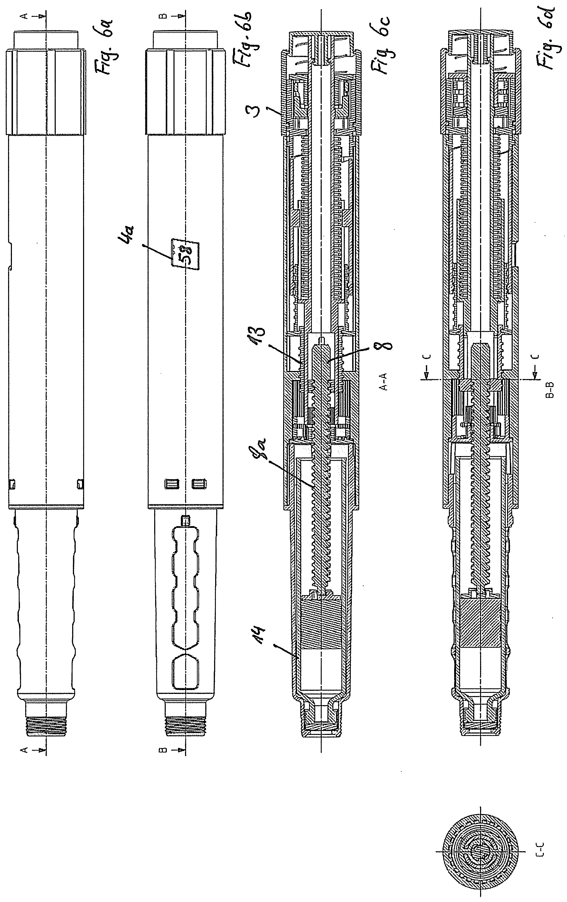

In den Figuren 6a-d wird der Zustand der Antriebs- und Dosiervorrichtung dargestellt, bei der in dem Produktbehälter 14 eine ausschüttbare Menge des Produkts enthalten ist, die geringer als die mit der Antriebs- und Dosiervorrichtung einstellbare Maximaldosis ist. In dem gezeigten Beispiel sind 58 IU in dem Produktbehälter 14 enthalten, wobei mit der Antriebs- und Dosiervorrichtung maximal 60 IU einstellbar wären. Um eine Fehlanwendung zu vermeiden, umfasst die Antriebs- und Dosiervorrichtung eine Begrenzungseinrichtung, welche die Dosiseinstellung begrenzt. Hierzu weist das Vortriebsglied 8 einen Anschlag am proximalen Ende des Gewindes 8a auf, an dem die Gewindehülse 13 anschlägt, wodurch eine Drehung des Dosierglieds 2 in die zweite Drehrichtung blockiert wird, auch wenn der Maximaldosisanschlag 10c nicht in dem Anschlag mit dem Maximaldosisgegenanschlag 15a ist. Eine Drehung des Dosierglieds 3 in die erste Drehrichtung ist jedoch möglich.

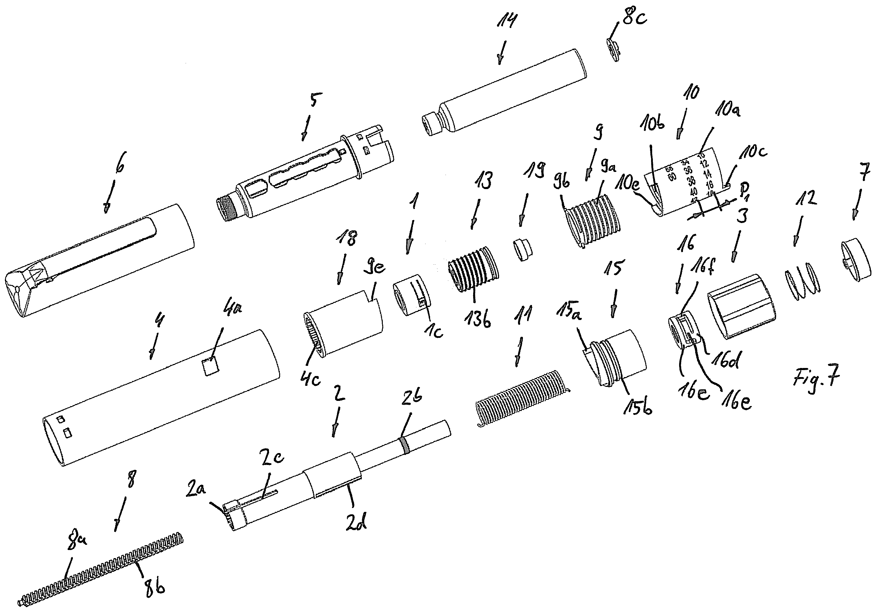

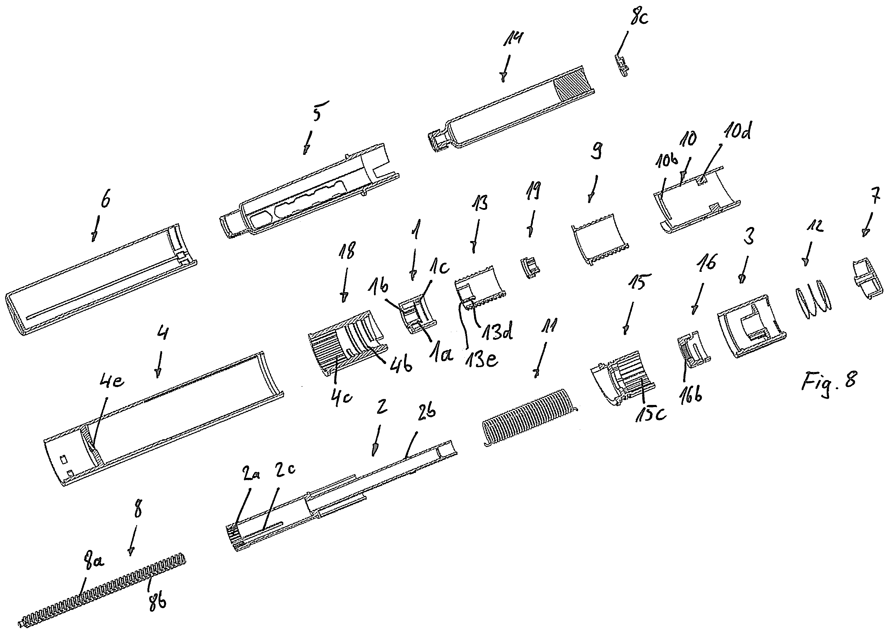

In der zweiten, in den Figuren 7-12d gezeigten Ausführungsform weist das Gehäuse 4 ein Innengewinde 4e auf, welches in das Außengewinde 8a des Vortriebsglieds 8 eingreift. In dem Gehäuse 4 ist ein weiterer Gehäuseeinsatz 18 angeordnet, an dessen Innenumfang die Verzahnung 4c und das in das Außengewinde der Gewindehülse 13 eingreifende Innengewinde 4b gebildet werden. Ferner bildet der Gehäuseeinsatz 18 den Nulldosisgegenanschlag 9e in der Gestalt eines in Umfangrichtung wirkenden Drehanschlags.

Das Rotationsglied 1 ist drehfest aber axial verschiebbar mit dem Vortriebsglied 8 verbunden. Ein Steg 1b greift hierfür in die Nut 8b des Vortriebsglieds 8. Im Gegensatz zur ersten Ausführungsform weist die Gewindehülse 13 kein Innengewinde auf sondern lediglich einen Führungsabschnitt, an dem sich die Gewindehülse 13 an den Gewindespitzen des Außengewindes 8a abstützt.

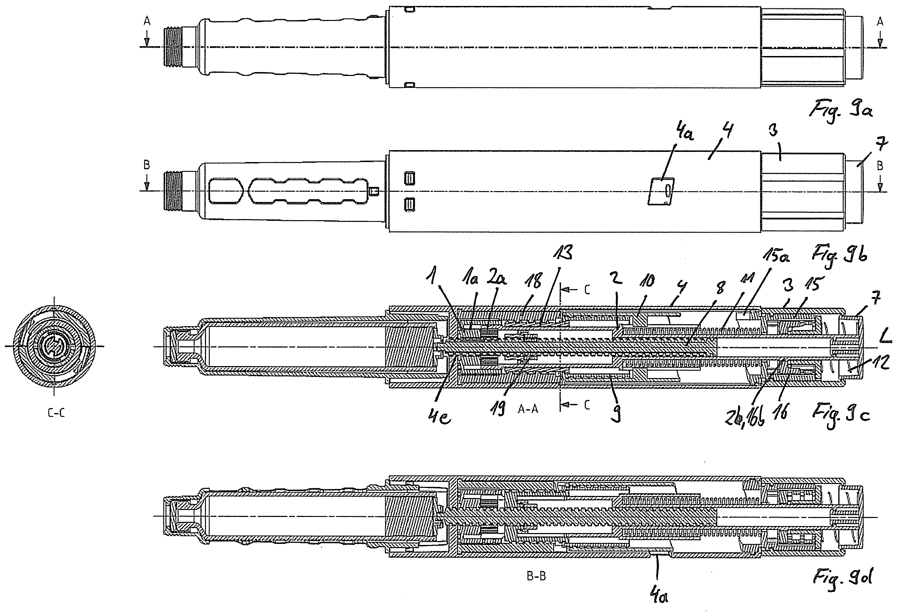

In den Figuren 9a bis 9d wird die Antriebs- und Dosiervorrichtung in dem Ausgangs- oder Auslieferungszustand gezeigt, wobei eine Dosis Null eingestellt ist. Durch Drehung des Dosierglieds in die zweite Drehrichtung wird über die geschlossene zweite Kupplung 2b, 16b das Kupplungsglied 2 relativ zu dem Gehäuse 4 in die zweite Drehrichtung gedreht, wobei das Dosisanzeigeelement 10 an dem Lagerelement 9 mit Hilfe des Gewindes 10b in proximale Richtung entlang geschraubt wird. Ferner wird die Gewindehülse 13 an dem Innengewinde 4b des weiteren Gehäuseeinsatzes 18 in proximale Richtung geschraubt, wodurch das drehfest an dem Gehäuse 4 längsgeführte Lagerelement 9 in proximale Richtung verschoben wird. Während der Dosiseinstellung ist die erste Kupplung 2a, 1a geöffnet, wobei das Kupplungsglied 2 relativ zu dem Vortriebsglied 8 gedreht wird.

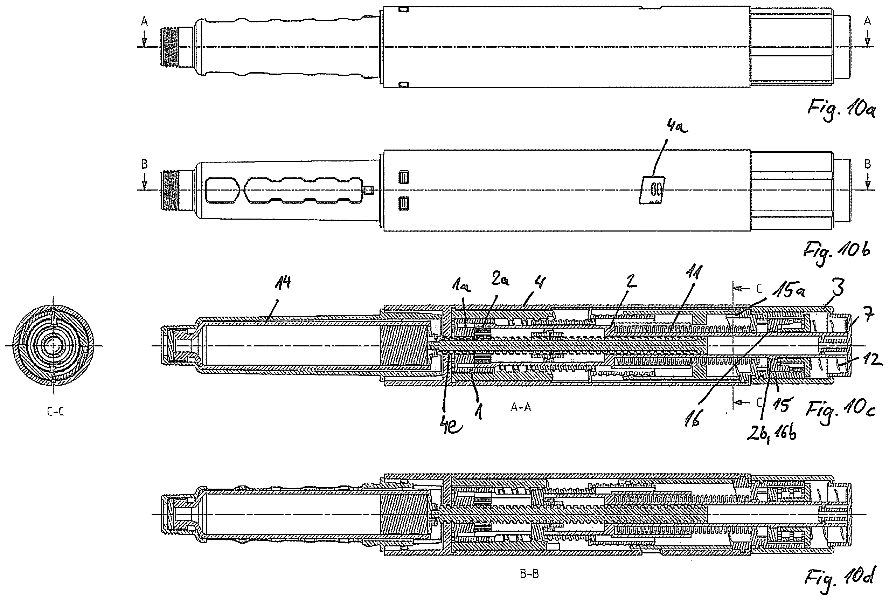

In den Figuren 10a-d ist die mit der Antriebs- und Dosiervorrichtung einstellbare Maximaldosis, in diesem Beispiel 60 IU, eingestellt, wobei der Maximaldosisanschlag 10c an dem Maximaldosisgegenanschlag 15a anschlägt. Selbstverständlich kann die Dosis durch Drehen des Dosierglieds 3 in die erste Drehrichtung verringert werden.

Zur Ausschüttung der eingestellten Produktdosis wird das Betätigungsglied 7 gedrückt, wodurch das Kupplungsglied 2 relativ zu dem Gehäuse 4 in distale Richtung verschoben wird, wobei die erste Kupplung 1a, 2a geschlossen und die zweite Kupplung 2b, 16b geöffnet wird.

Das Kupplungsglied 2 weist insbesondere in dem Bereich, an dem die Kupplungsstruktur 2a gebildet wird, einen Kragen auf, der im unbetätigten Zustand das Eingriffsglied 1c in dem Eingriff mit der Verzahnung 4c hält, wodurch verhindert wird, dass das Rotationsglied 1 und somit die Kolbenstange 8 relativ zu dem Gehäuse 4 verdreht wird. Durch Betätigung des Betätigungsglieds 7 wird außerdem das Eingriffsglied 1c freigegeben, nämlich der Kragen aus dem Eingriff mit dem Eingriffsglied 1c bewegt. Durch Öffnen der zweiten Kupplung 2b, 16b wird das Kupplungsglied 2 von der Antriebsfeder 11 in die erste Drehrichtung gedreht, wodurch das Rotationsglied 1, welches drehfest aber axial verschiebbar in das Vortriebsglied 8 eingreift, relativ zu dem Gehäuse 4 verdreht wird, und das Vortriebsglied 8 mitdreht. Das Vortriebsglied 8 schraubt sich mit seinem Außengewinde an dem Innengewinde 4e des Gehäuses 4 in distale Richtung, wodurch der Kolben in den Produktbehälter 14 verschoben wird.

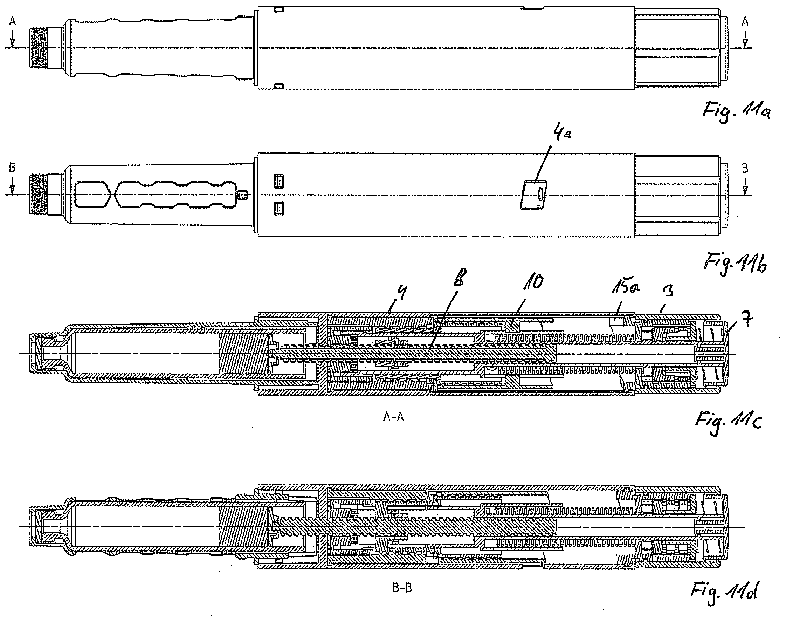

Bei der Produktausschüttung dreht sich das Dosisanzeigeelement 10 in seine Nulldosisposition zurück, d.h. der Nulldosisanschlag 10e wird zu dem Nulldosisgegenanschlag 9e hin bewegt, wobei die Dosisskala 10a in der Zeigeeinrichtung 4a in Richtung Null herunterzählt. Die Ausschüttung ist beendet, wenn in der Zeigeeinrichtung 4a die Dosis Null erscheint oder/und der Nulldosisanschlag 10e an dem Nulldosisgegenanschlag 9e anschlägt. In den Figuren 11a-d wird die Antriebs- und Dosiervorrichtung nach der erfolgten Produktausschüttung gezeigt, wobei das Betätigungsglied 7 noch betätigt ist.

Durch wiederholtes Dosieren und Betätigen des Betätigungsglieds 7 kann das in dem Produktbehälter 14 enthalten Produkt mit mehreren frei wählbaren Dosen ausgeschüttet werden.

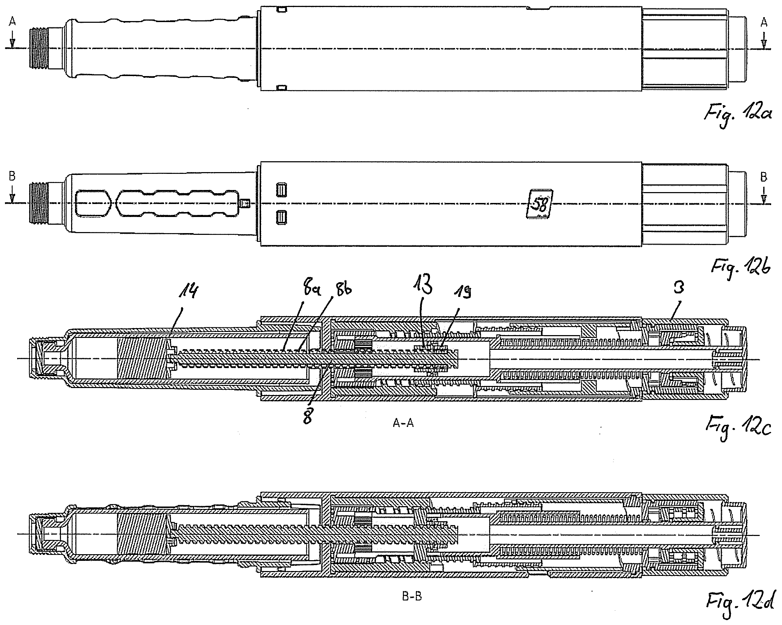

In den Figuren 12a-d wird die Antriebs- und Dosiervorrichtung in dem Zustand gezeigt, der sinngemäß dem Zustand aus den Figuren 6a-d entspricht. Um eine Drehung des Dosierglieds 3 in die zweite Drehrichtung zu verhindern, ist eine Zwischenhülse 19 zwischen der Gewindehülse 13 und einem Stoppanschlag des Vortriebsglieds 8 angeordnet. Die Zwischenhülse 19 kann z. B. in die Nut 8b eingreifen, wobei z. B. das proximale Ende der Nut 8b den Stoppanschlag bilden kann. Alternativ kann die Zwischenhülse 19 mit einem Innengewinde in das Außengewinde 8a eingreifen, wobei der Stoppanschlag am Ende eines Gewindegangs gebildet werden kann.

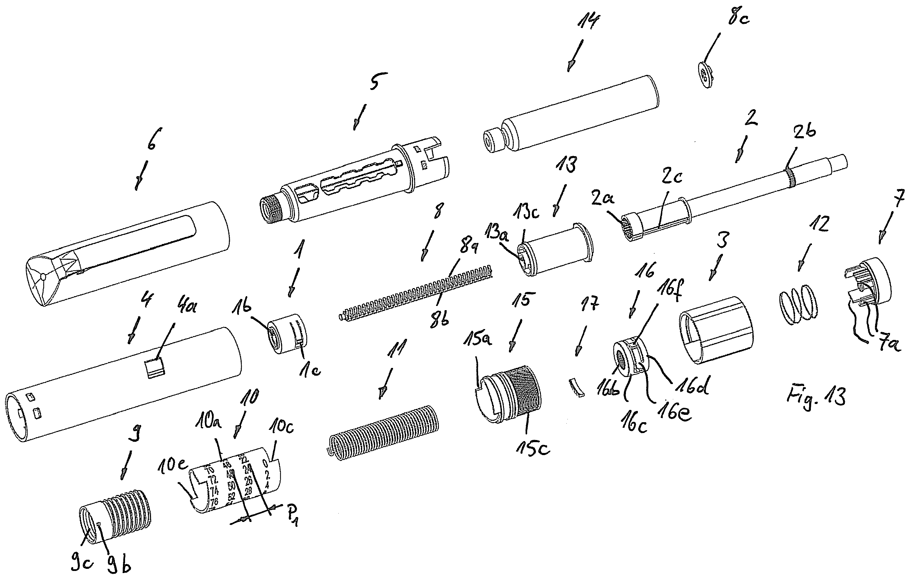

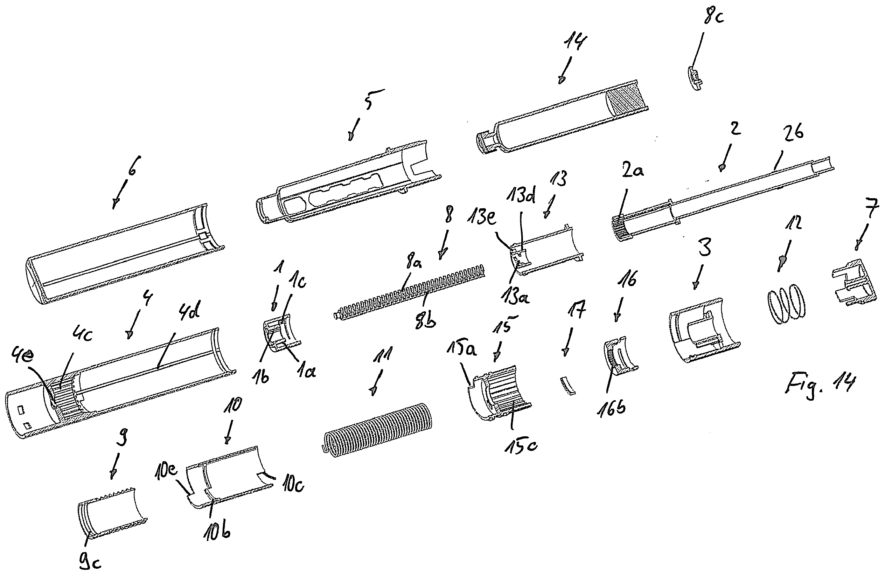

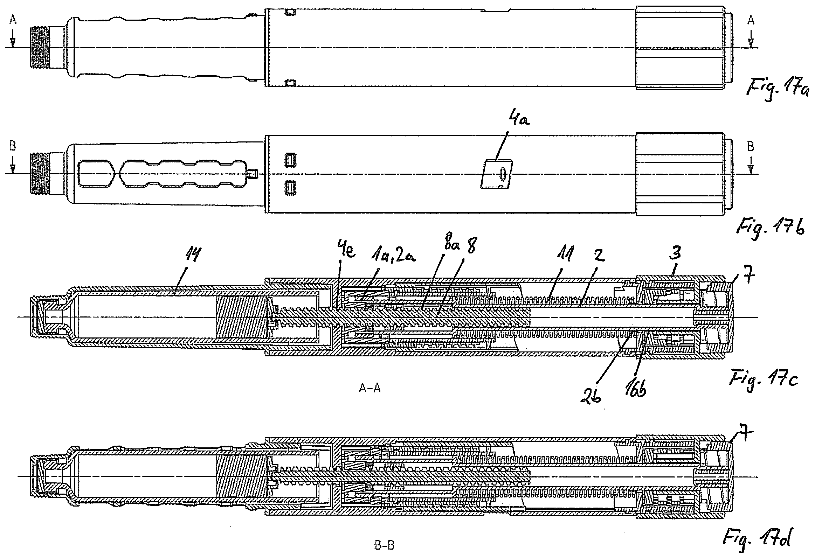

Die dritte Ausführungsform aus den Figuren 13-18d weist eine Gewindehülse 13 auf, die mit ihrem Innengewinde 13a in das Außengewinde 8a des Vortriebsglieds 8 eingreift und insbesondere kein Außengewinde aufweist. Ferner weist das Gehäuse 4 ein Innengewinde 4e auf, welches in das Außengewinde 8a des Vortriebsglieds 8 eingreift, so dass eine Drehbewegung des Vortriebsglieds 8, insbesondere in die erste Drehrichtung, eine Bewegung des Vortriebsglieds 8 in Ausschüttrichtung, d. h. in distale Richtung bewirkt. Der Gehäuseeinsatz 15 weist ferner ein Außengewinde 15c auf, in welches ein Begrenzer 17 in der Gestalt eines Kreissegments eingreift. Der Begrenzer 17 weist an seiner konkaven Fläche ein Gewinde auf, welches mit dem Gewinde 15c in einem Eingriff ist. Der Begrenzer 17 ist mit dem Dosierglied 3 drehfest verbunden, so dass der Begrenzer 17 mit dem Dosierglied 3 um den Gehäuseeinsatz 15 herum mitgedreht wird.

Der Begrenzer 17 dient zur Verhinderung der Einstellung einer Dosis, welche die in dem Produktbehälter 14 enthaltene ausschüttbare Produktdosis übersteigt.

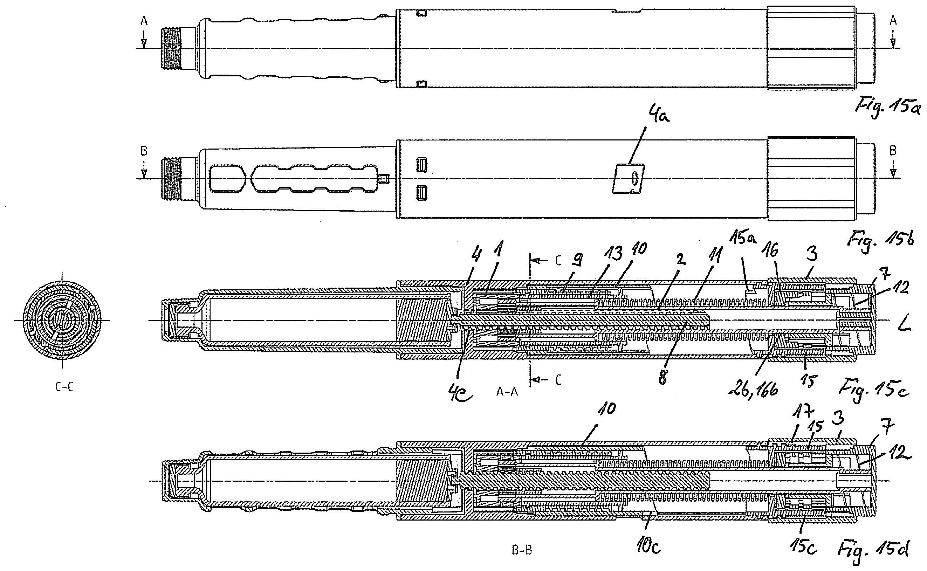

In den Figuren 15a bis 15d wird die Antriebs- und Dosiervorrichtung in dem Ausgangs- oder Auslieferungszustand gezeigt. Zur Einstellung der Produktdosis wird das Dosierglied 3 relativ zu dem Gehäuse 4 verdreht, wodurch über die geschlossene zweite Kupplung 2b, 16b das Kupplungsglied 2 relativ zu dem Gehäuse 4 verdreht wird. Das Kupplungsglied 2 dreht die Gewindehülse 13 mit, die sich dadurch an dem Vortriebsglied 8 in englang der Längsachse L schraubt. Die Gewindehülse 13 ist ferner mittels Abragung und Längsführungsnut (nicht gezeigt) in einem drehfesten aber axial verschiebbaren Eingriff mit dem Dosisanzeigeelement 10, wodurch das Dosisanzeigeelement 10 mit dem Kupplungsglied 2 und der Gewindehülse 13 mitgedreht wird. Die Gewindehülse 13 nimmt ferner das drehfest und axial verschiebbar an dem Gehäuse 4 geführte Lagerelement 9 entlang der Längsachse L mit.

Ferner weist das Kupplungsglied 2 im Bereich der Kupplungsstruktur 2a den im Zusammenhang mit der zweiten Ausführungsform beschriebenen Kragen auf, welcher das Eingriffsglied 1c in dem Eingriff mit der Verzahnung 4c hält, wenn das Betätigungsglied 7 unbetätigt ist und freigibt, wenn das Betätigungsglied 7 betätigt ist. Somit ist das Rotationsglied 1 und die Kolbenstange 8 während der Dosiseinstellung in Bezug auf das Gehäuse 4 verdrehgesichert.

Bei der Dosiseinstellung, insbesondere bei der Drehung des Dosierglieds 3 in die zweite Drehrichtung, d. h. zur Dosiserhöhung wird der Begrenzer 17 zu einem Stoppanschlag hin, der von dem Dosierglied 3 gebildet wird, bewegt. Der Abstand zu dem Stoppanschlag ist proportional zu der in dem Produktbehälter 14 enthaltenen ausschüttbaren Produktmenge. Wird das Dosierglied 3 in die erste Drehrichtung gedreht, wird der Begrenzer 17 von dem Stoppanschlag wegbewegt.

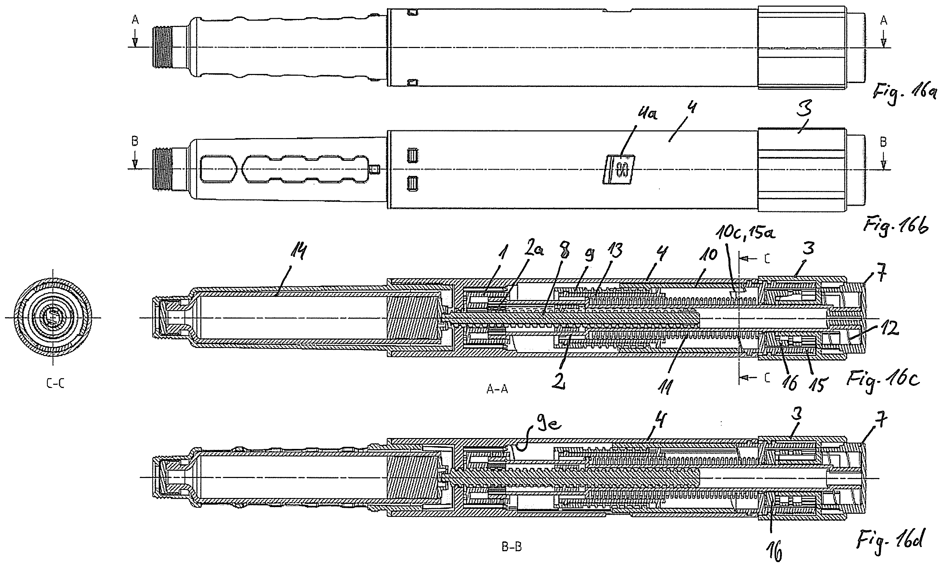

In den Figuren 16a-d wird die Antriebs- und Dosiervorrichtung mit einer maximal eingestellten Dosis, hier 80 IU, gezeigt, wobei der Maximaldosisanschlag 10c an dem Maximaldosisgegenanschlag 15a anschlägt. Durch Drehung des Dosierglieds 3 in die erste Drehrichtung kann die eingestellte Dosis natürlich verringert werden.

Zum Ausschütten der eingestellten Dosis wird das Betätigungsglied 7 gedrückt, wodurch die erste Kupplung 1a, 2a geschlossen und die zweite Kupplung 2b, 16b geöffnet wird und die Feder 11 das Kupplungsglied 2 in die erste Drehrichtung dreht. Aufgrund der geschlossenen ersten Kupplung 1a, 2a dreht das Kupplungsglied 2 das Rotationsglied 1 mit, welches wiederum das Vortriebsglied 8 mitdreht, wodurch das Vortriebsglied 8 mit seinem Außengewinde 8a an dem Gewinde 4e des Gehäuses 4 in distale Richtung geschraubt wird und dabei den Kolben in dem Produktbehälters 14 verschiebt. Während der Dosisausschüttung schraubt sich das Dosisanzeigeelement 10 zurück in Richtung Nulldosisposition. Die Ausschüttung ist beendet, wenn der Nulldosisanschlag 10e des Dosisanzeigeelements 10 an den Nulldosisgegenanschlag 9e, der von dem Gehäuse 4 gebildet wird, anschlägt oder/und in der Zeigeeinrichtung 4a die Dosis Null erscheint.

Das Dosierglied 3 dreht sich während der Produktausschüttung relativ zu dem Gehäuse 4 nicht, da der Betätigungsknopf 7 mittels Abragungen/Eingriffsgliedern 7a in die Verzahnung 15c eingekuppelt ist. Dadurch hält der Begrenzer 17 seine Position in Bezug auf den Stoppanschlag bei, wodurch insbesondere ein Zählwerk gebildet wird, welches bei der Dosiseinstellung zählt und bei der Dosisausschüttung nicht zählt.

Durch wiederholtes Dosieren und Betätigen des Betätigungsglieds 7 kann das in dem Produktbehälter 14 enthaltene Produkt in mehreren frei wählbaren Einzeldosen ausgeschüttet werden.

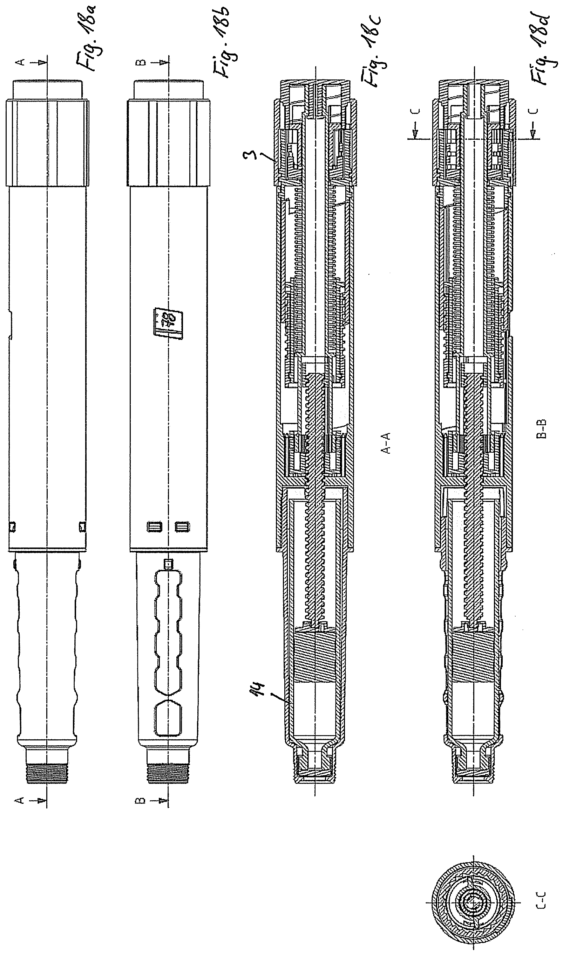

In den Figuren 18a-d wird die Antriebs- und Dosiervorrichtung in einem Zustand gezeigt, in der die maximal einstellbare Dosis die in dem Produktbehälter 14 enthaltene Produktmenge übersteigt. Um zu verhindern, dass eine größere als aus dem Produktbehälter 14 ausschüttbare Produktmenge ausgeschüttet werden kann, hat sich der Begrenzer im Laufe der einzelnen Dosierungen soweit an den Stoppanschlag heran bewegt, dass er an dem Stoppanschlag anschlägt, wodurch eine Drehung des Dosierglieds 3 relativ zu dem Gehäuse 4 in eine zweite Drehrichtung verhindert wird. Eine Drehung des Dosierglieds 3 in die erste Drehrichtung ist jedoch noch möglich.

**Bezugszeichenliste**

| | | | |
|---|---|---|---|
| 1 | Rotationsglied | 10b | Gewinde / Innengewinde |
| 1a | zweite Kupplungsstruktur | 10c | Maximaldosisanschlag |
| 1b | Steg | 10d | Eingriffsnocken/Abragung |
| 1c | Eingriffsglied / Nocken | 10e | Nulldosisanschlag |
| | | | |
| 2 | Kupplungsglied/erstes Drehglied | 11 | Feder / Ausschüttfeder / Drehfeder |
| 2a | erste Kupplungsstruktur/Innenverzahnung | | |
| 2b | dritte Kupplungsstruktur | 12 | Kupplungsfeder / Rücksetzfeder |
| 2c | Führungsschlitz | | |
| 2d | Führung / Längsführung | 13 | Gewindehülse |
| | | 13a | Innengewinde/Gewinde |
| 3 | Dosierglied/Aufziehglied/zweites Drehglied | 13b | Außengewinde/Gewinde |
| 3a | Abragung / Längssteg | 13c | Ringnut |
| 3b | Ringsteg | 13d | Innenhülse |
| | | 13e | Steg |
| 4 | Gehäuse | | |
| 4a | Zeigeeinrichtung | 14 | Produktbehälter |
| 4b | Gewinde / Innengewinde | | |
| 4c | Verzahnung/Innenverzahnung | 15 | Gehäuseeinsatz/erste Hülse/Ratschenhülse |
| 4d | Längsführung / Nut | 15a | Maximaldosisgegenaschlag |
| 4e | Innengewinde | 15b | Ringnut |
| | | 15c | Innenverzahnung/Verzahnung |
| 5 | Produktbehälter | | |
| | | 16 | Kupplungselement / 2-Wege-Kupplung |
| 6 | Kappe | 16a | Anschlag/Kante/Nut |
| 7 | Betätigungsglied / Betätigungsknopf | 16b | Verdrehsicherung / vierte Kupplungsstruktur |
| 7a | Abragungen | 16c | erster Abschnitt |
| | | 16d | zweiter Abschnitt |
| 8 | Vortriebsglied / Stößel / Kolbenstange | 16e | Zwischenabschnitt |
| 8a | Außengewinde | 16f | Eingriffsglied/erste Zahnflanke/zweite Zahnflanke |
| 8b | Längsführung / Nut | 16g | Anschlag/Kante |
| 8c | Flansch | | |
| | | 17 | Begrenzer |
| 9 | Lagerelement | | |
| 9a | Gewinde/Außengewinde | 18 | Gehäuseeinsatz |
| 9b | Abragung, | | |
| 9c | Ringsteg | 19 | Zwischenhülse |
| 9e | Nulldosisgegenanschlag | | |
| | | L | Längsachse / Drehachse |
| 10 | Dosisanzeigeelement | | |
| 10a | Dosisskala | P₁ | Steigung der wendelförmigen Skala |

## Patentansprüche

1. Antriebs- und Dosiervorrichtung für eine Injektionsvorrichtung zur Verabreichung eines flüssigen Produkts, insbesondere eines Medikaments, wobei mit der Antriebs- und Dosiervorrichtung eine zu verabreichende Produktdosis einstellbar ist, umfassend:
a) ein in Bezug auf ein Kupplungsglied (2) drehfestes Dosisanzeigeelement (10), über dessen Umfang eine wendelförmige Dosisskala (10a) angeordnet ist,
b) eine Zeigeeinrichtung (4a) und ein vom Verwender der Antriebs- und Dosiervorrichtung greifbares Dosierglied (3), wobei zur Einstellung der zu verabreichenden Dosis mittels Drehung des Dosierglieds (3) relativ zu der Zeigeeinrichtung (4a) das Dosisanzeigeelement (10) relativ zu der Zeigeeinrichtung (4a) um eine Drehachse (L) schraubbar und relativ zu dem Dosierglied (3) entlang der Drehachse (L) bewegbar ist, wobei mittels der Zeigeeinrichtung (4a) ein Wert der Dosisskala (10a) ablesbar ist, welcher der eingestellten Dosis entspricht,
c) ein Lagerelement (9), welches ein Gewinde (9a) aufweist, welches mit einem Gewinde (10b) des Dosisanzeigeelements (10) in einem Gewindeeingriff ist,
**dadurch gekennzeichnet**,
d) dass die Gewindesteigung des Gewindes (10b) des Dosisanzeigeelements (10) ungleich der Steigung (P₁) der wendelförmigen Skala (10a) ist, und
e) dass die Antriebs- und Dosiervorrichtung ein Gehäuse (4) und eine Gewindehülse (13) umfasst, welche drehbar und axialfest mit dem Lagerelement (9) sowie drehfest und axial verschiebbar mit dem Kupplungsglied (2) verbunden ist, und ein Gewinde (13a, 13b) aufweist zum Eingriff in ein beim Einstellen der zu verabreichenden Dosis gegenüber dem Gehäuse (4) axialfestes Gewinde (8a, 4b).

2. Antriebs- und Dosiervorrichtung nach Anspruch 1, **dadurch gekennzeichnet, dass** das Lagerelement (9) relativ zu dem Gehäuse (4) bewegbar, insbesondere drehfest und axial verschiebbar ist.

3. Antriebs- und Dosiervorrichtung nach einem der vorhergehenden Ansprüche, **dadurch gekennzeichnet, dass** das Gewinde (13a, 13b) der die Gewindehülse (13) insbesondere ein Innengewinde (13a) ist, und das axialfeste Gewinde ein Gewinde (8a) eines Vortriebsglieds (8) ist.

4. Antriebs- und Dosiervorrichtung nach einem der vorhergehenden Ansprüche 1 oder 2, **dadurch gekennzeichnet, dass** die Gewindehülse (13) ein Gewinde, insbesondere Außengewinde (13b), welches in ein gehäusefestes Gewinde (4b) eingreift, aufweist.

5. Antriebs- und Dosiervorrichtung nach Anspruch 3 oder 4, **dadurch gekennzeichnet, dass**
- die Summe aus der Gewindesteigung des in das Gewinde (9a) des Lagerelements (9) eingreifenden Gewindes (10b) des Dosisanzeigeelements (10) und der Gewindesteigung des in das Gewinde (8a) des Vortriebsglieds (8) eingreifenden Gewindes (13a) der Gewindehülse (13) oder/und
- die Summe aus der Gewindesteigung des in das Gewinde (9a) des Lagerelements (9) eingreifenden Gewindes (10b) des Dosisanzeigeelements (10) und i) der Gewindesteigung des in das gehäusefeste Gewinde (4b) eingreifenden Gewindes (13b) der Gewindehülse (13) oder ii) der Gewindesteigung des in ein gehäusefestes Gewinde (4e) eingreifenden Gewindes (8a) des Vortriebsglieds (8)
gleich der Steigung (P₁) der wendelförmigen Skala (10a) des Dosisanzeigeelements (10) ist.

6. Antriebs- und Dosiervorrichtung nach Anspruch 3, **dadurch gekennzeichnet, dass** die Gewindehülse (13) während der Dosiseinstellung relativ zu dem Vortriebsglied (8) drehbar und während der Produktausschüttung relativ zu dem Vortriebsglied (8) drehfest ist.

7. Antriebs- und Dosiervorrichtung nach einem der vorhergehenden Ansprüche, **dadurch gekennzeichnet, dass** das Dosisanzeigeelement (10) drehfest und axialverschiebbar mit einem Kupplungsglied (2), welches während Dosiseinstellung und vorzugsweise während der Produktausschüttung relativ zu der Zeigeeinrichtung (4a) drehbar ist, verbunden ist.

8. Antriebs- und Dosiervorrichtung nach einem der vorhergehenden Ansprüche, **gekennzeichnet durch** ein für die Produktausschüttung betätigbares Betätigungsglied (7) und eine, insbesondere erste, Kupplung (1a, 2a), welche bei unbetätigtem Betätigungsglied (7) geöffnet und bei betätigtem Betätigungsglied (7) geschlossen ist oder **durch** Betätigung des Betätigungsglieds (7) geschlossen wird, wobei die geschlossene Kupplung (1a, 2a) das Kupplungsglied (2) und das Vortriebsglied (8) drehfest miteinander verbindet, wobei das Kupplungsglied (2) bei geöffneter Kupplung (1a,2a) relativ zu dem Vortriebsglied (8) drehbar ist.

9. Antriebs- und Dosiervorrichtung nach einem der vorhergehenden Ansprüche, **gekennzeichnet durch** ein für die Produktausschüttung betätigbares Betätigungsglied (7) und eine, insbesondere zweite Kupplung (2b, 16b), welche bei unbetätigtem Betätigungsglied (7) geschlossen und bei betätigtem Betätigungsglied (7) geöffnet ist oder **durch** Betätigung des Betätigungsglieds (7) geöffnet wird, wobei die geschlossene Kupplung (2b, 16b) das Dosierglied (3) und das Kupplungsglied (2) zumindest in eine Drehrichtung drehfest miteinander verbindet, wobei das Kupplungsglied (2) bei geöffneter Kupplung (2b, 16b) relativ zu dem Dosierglied (3) drehbar ist.

10. Antriebs- und Dosiervorrichtung nach wenigstens einem der zwei vorhergehenden Ansprüche, **gekennzeichnet durch** eine Kupplungsfeder (12), welche die erste Kupplung (1a, 2a) in ihre geöffneten Stellung und/oder die zweite Kupplung (2b, 16b) in ihre geschlossenen Stellung spannt oder drückt, wenn das Betätigungsglied (7) unbetätigt ist.

11. Antriebs- und Dosiervorrichtung nach einem der vorhergehenden Ansprüche, **gekennzeichnet durch** eine Antriebsfeder (11), welche vorgespannt ist oder mittels Drehung des Dosierglieds (3) vorspannbar ist und das Vortriebsglied (8) während der Produktausschüttung antreibt.

12. Antriebs- und Dosiervorrichtung nach dem vorhergehenden Anspruch, **gekennzeichnet durch** eine lösbare Sperreinrichtung (16), welche so zwischen das Dosierglied (3) und das Kupplungsglied (2) geschaltet ist, dass das von der Antriebsfeder (11) auf das Kupplungsglied (2) ausgeübte Drehmoment in das Gehäuse (4) geleitet wird, so dass das Kupplungsglied (2) relativ zu dem Gehäuse (4) verdrehgesichert ist, wobei diese Verdrehsicherung des Kupplungsglieds (2) mittels Drehung des Dosierglieds (3) lösbar ist, so dass das Kupplungsglied (2) relativ zu dem Gehäuse (4) in eine erste und vorzugsweise auch in eine zweite Drehrichtung drehbar ist.

## Claims

1. A drive and dosing device for an injection device for administering a liquid product, in particular a medicament, wherein a product dose to be administered can be set with the drive and dosing device, comprising:
a) a dose indicator element (10) which is non-rotatable with respect to a coupling member (2) and over the periphery of which is arranged a helical dose scale (10a),
b) an indicator device (4a) and a dosing member (3) which can be gripped by the user of the drive and dosing device, wherein for setting the dose to be administered by means of rotation of the dosing member (3) relative to the indicator device (4a) the dose indicator element (10) can be screwed relative to the indicator device (4a) about an axis of rotation (L) and is movable relative to the dosing member (3) along the axis of rotation (L), wherein a value of the dose scale (10a) which corresponds to the set dose can be read by means of the indicator device (4a),
c) a bearing element (9) having a thread (9a) which is in threaded engagement with a thread (10b) of the dose indicator element (10),
**characterised in that**
d) the thread pitch of the thread (10b) of the dose indicator element (10) is not equal to the pitch (P₁) of the helical scale (10a), and
e) the drive and dosing device includes a housing (4) and a threaded sleeve (13) which is connected rotatably and axially fixedly to the bearing element (9) and non-rotatably and axially displaceably to the coupling member (2), and has a thread (13a, 13b) for engagement into a thread (8a, 4b) which is axially fixed in relation to the housing (4) upon setting of the dose to be administered.

2. A drive and dosing device according to claim 1 **characterised in that** the bearing element (9) is movable, in particular non-rotatably and axially displaceable, relative to the housing (4).

3. A drive and dosing device according to one of the preceding claims **characterised in that** the thread (13a, 13b) of the threaded sleeve (13) is in particular an internal thread (13a) and the axially fixed thread is a thread (8a) of an advance member (8).

4. A drive and dosing device according to one of preceding claims 1 and 2 **characterised in that** the threaded sleeve (13) has a thread, in particular an external thread (13b), which engages in a thread (4b) which is fixed with respect to the housing.

5. A drive and dosing device according to claim 3 or claim 4 **characterised in that**
- the sum of the thread pitch of the thread (10b) of the dose indicator element (10), that engages into the thread (9a) of the bearing element (9), and the thread pitch of the thread (13a) of the threaded sleeve (13), that engages into the thread (8a) of the advance member (8), and/or
- the sum of the thread pitch of the thread (10b) of the dose indicator element (10), that engages into the thread (9a) of the bearing element (9), and i) the thread pitch of the thread (13b) of the threaded sleeve (13), that engages into the thread (4b) which is fixed with respect to the housing, or ii) the thread pitch of the thread (8a) of the advance member (8), that engages into a thread (4e) which is fixed with respect to the housing,
is equal to the pitch (P₁) of the helical scale (10a) of the dose indicator element (10).

6. A drive and dosing device according to claim 3 **characterised in that** the threaded sleeve (13) is rotatable relative to the advance member (8) during dose setting and is non-rotatable relative to the advance member (8) during product discharge.

7. A drive and dosing device according to one of the preceding claims **characterised in that** the dose indicator element (10) is connected non-rotatably and axially displaceable to a coupling member (2) which is rotatable relative to the indicator device (4a) during dose setting and preferably during product discharge.

8. A drive and dosing device according to one of the preceding claims **characterised by** an actuating member (7) which is actuable for product discharge and a, in particular first, coupling (1a, 2a), which is open when the actuating member (7) is unactuated and is closed when the actuating member (7) is actuated or becomes closed by actuation of the actuating member (7), wherein the closed coupling (1a, 2a) connects the coupling member (2) and the advance member (8) together non-rotatably, wherein the coupling member (2) is rotatable relative to the advance member (8) when the coupling (1a, 2a) is open.

9. A drive and dosing device according to one of the preceding claims **characterised by** an actuating member (7) which is actuable for product discharge and a, in particular second, coupling (2b, 16b), which is closed when the actuating member (7) is unactuated and is open when the actuating member (7) is actuated or becomes opened by actuation of the actuating member (7), wherein the closed coupling (2b, 16b) connects the dosing member (3) and the coupling member (2) together non-rotatably at least in one direction of rotation, wherein the coupling member (2) is rotatable relative to the dosing member (3) when the coupling (2b, 16b) is open.

10. A drive and dosing device according to at least one of the two preceding claims **characterised by** a coupling spring (12) which stresses or presses the first coupling (1a, 2a) into its open position and/or the second coupling (2b, 16b) into its closed position when the actuating member (7) is unactuated.

11. A drive and dosing device according to one of the preceding claims **characterised by** a drive spring (11) which is biased or can be biased by means of rotation of the dosing member (3) and drives the advance member (8) during product discharge.

12. A drive and dosing device according to the preceding claim **characterised by** a releasable locking device (16) which is connected between the dosing member (3) and the coupling member (2) in such a way that the torque exerted by the drive spring (11) on the coupling member (2) is transmitted into the housing (4) so that the coupling member (2) is secured against rotation relative to the housing (4), wherein said rotational securing action of the coupling member (2) is releasable by means of rotation of the dosing member (3) so that the coupling member (2) is rotatable relative to the housing (4) in a first and preferably also in a second direction of rotation.

## Revendications

1. Dispositif d'entraînement et de dosage pour un dispositif d'injection servant à administrer un produit liquide, en particulier un médicament, dans lequel une dose de produit à administrer peut être réglée avec le dispositif d'entraînement et de dosage, comprenant :
a) un élément d'affichage de dose (10) solidaire en rotation par rapport à un organe de couplage (2), sur le périphérique duquel une graduation de dosage (10a) hélicoïdale est disposée,
b) un système indicateur (4a) et un organe de dosage (3) pouvant être saisi par l'utilisateur du dispositif d'entraînement et de dosage, dans lequel pour régler la dose à administrer, une rotation de l'organe de dosage (3) par rapport au système indicateur (4a) permet de visser l'élément d'affichage de dose (10) par rapport au système indicateur (4a) autour d'un axe de rotation (L) et de le déplacer par rapport à l'organe de dosage (3) le long de l'axe de rotation (L), dans lequel le système indicateur (4a) permet de lire une valeur de la graduation de dosage (10a), laquelle correspond à la dose réglée,
c) un élément de palier (9), lequel présente un filetage (9a), lequel est en prise par filetage avec un filetage (10b) de l'élément d'affichage de dose (10),
caractérisé en ce
d) que le pas de filetage du filetage (10b) de l'élément d'affichage de dose (10) n'est pas égal au pas (P₁) de la graduation (10a) hélicoïdale, et
e) que le dispositif d'entraînement et de dosage comprend un boîtier (4) et un manchon fileté (13), lequel est relié de manière solidaire en rotation et de manière solidaire axialement à l'élément de palier (9) ainsi que de manière solidaire en rotation et de manière à pouvoir coulisser axialement à l'organe de couplage (2), et présente un filetage (13a, 13b) destiné à venir en prise avec un filetage (8a, 4b) solidaire axialement par rapport au boîtier (4) lors du réglage de la dose à administrer.

2. Dispositif d'entraînement et de dosage selon la revendication 1, **caractérisé en ce que** l'élément de palier (9) peut être déplacé, en particulier peut être coulissé de manière solidaire en rotation et axialement, par rapport au boîtier (4).

3. Dispositif d'entraînement et de dosage selon l'une quelconque des revendications précédentes, **caractérisé en ce que** le filetage (13a, 13b) est le manchon fileté (13), en particulier un filetage intérieur (13a), et le filetage solidaire axialement est un filetage (8a) d'un organe de propulsion (8).

4. Dispositif d'entraînement et de dosage selon l'une quelconque des revendications 1 ou 2, **caractérisé en ce que** le manchon fileté (13) présente un filetage, en particulier un filetage extérieur (13b), lequel vient en prise avec un filetage (4b) solidaire du boîtier.

5. Dispositif d'entraînement et de dosage selon la revendication 3 ou 4, **caractérisé en ce que**
- la somme du pas de filetage du filetage (10b), venant en prise avec le filetage (9a) de l'élément de palier (9), de l'élément d'affichage de dosage (10) et du pas de filetage du filetage (13a), venant en prise avec le filetage (8a) de l'organe de propulsion (8), du manchon fileté (13) et/ou
- la somme du pas de filetage du filetage (10b), venant en prise avec le filetage (9a) de l'élément de palier (9), de l'élément d'affichage de dose (10) et i) du pas de filetage du filetage (13b), venant en prise avec le filetage (4b) solidaire du boîtier, du manchon fileté (13) ou ii) du pas de filetage du filetage (8a), venant en prise avec un filetage (4e) solidaire du boîtier, de l'organe de propulsion (8) est égale au pas (P₁) de la graduation (10a) hélicoïdale de l'élément d'affichage de dose (10).

6. Dispositif d'entraînement et de dosage selon la revendication 3, **caractérisé en ce que** le manchon fileté (13) peut tourner pendant le réglage de dose par rapport à l'organe de propulsion (8) et est solidaire en rotation pendant le déversement de produit par rapport à l'organe de propulsion (8).

7. Dispositif d'entraînement et de dosage selon l'une quelconque des revendications précédentes, **caractérisé en ce que** l'élément d'affichage de dose (10) est relié de manière solidaire en rotation et de manière à pouvoir être coulissé axialement à un organe de couplage (2), lequel peut tourner par rapport au système indicateur (4a) pendant le réglage de dose et de préférence pendant le déversement de produit.

8. Dispositif d'entraînement et de dosage selon l'une quelconque des revendications précédentes, **caractérisé par** un organe d'actionnement (7) pouvant être actionné pour le déversement de produit et un couplage (1a, 2a), en particulier un premier couplage, lequel est ouvert lorsque l'organe d'actionnement (7) n'est pas actionné et est fermé lorsque l'organe d'actionnement (7) est actionné ou est fermé par l'actionnement de l'organe d'actionnement (7), dans lequel le couplage (1a, 2a) fermé relie l'un à l'autre de manière solidaire en rotation l'organe de couplage (2) et l'organe de propulsion (8), dans lequel l'organe de couplage (2) peut être tourné par rapport à l'organe de propulsion (8) lorsque le couplage (1a, 2a) est ouvert.

9. Dispositif d'entraînement et de dosage selon l'une quelconque des revendications précédentes, **caractérisé par** un organe d'actionnement (7) pouvant être actionné pour le déversement de produit et un couplage (2b, 16b), en particulier un deuxième couplage, lequel est fermé lorsque l'organe d'actionnement (7) n'est pas actionné et est ouvert lorsque l'organe d'actionnement (7) est actionné ou est ouvert par l'actionnement de l'organe d'actionnement (7), dans lequel le couplage (2b, 16b) fermé relie l'un à l'autre de manière solidaire en rotation au moins dans une direction de rotation l'organe de dosage (3) et l'organe de couplage (2), dans lequel l'organe de couplage (2) peut tourner par rapport à l'organe de dosage (3) lorsque le couplage (2b, 16b) est ouvert.

10. Dispositif d'entraînement et de dosage selon au moins l'une quelconque des deux revendications précédentes, **caractérisé par** un ressort de couplage (12), lequel serre ou pousse le premier couplage (1a, 2a) dans sa position ouverte et/ou le deuxième couplage (2b, 16b) dans sa position fermée quand l'organe d'actionnement (7) n'est pas actionné.

11. Dispositif d'entraînement et de dosage selon l'une quelconque des revendications précédentes, **caractérisé par** un ressort d'entraînement (11), lequel est précontraint ou peut être précontraint au moyen d'une rotation de l'organe de dosage (3) et entraîne l'organe de propulsion (8) pendant le déversement de produit.

12. Dispositif d'entraînement et de dosage selon la revendication précédente, **caractérisé par** un système de fermeture (16) amovible, lequel est commuté de telle sorte entre l'organe de dosage (3) et l'organe de couplage (2) que le couple de rotation exercé par le ressort d'entraînement (11) sur l'organe de couplage (2) est conduit dans le boîtier (4) si bien que l'organe de couplage (2) est bloqué en rotation par rapport au boîtier (4), dans lequel ledit blocage en rotation de l'organe de couplage (2) peut être défait au moyen d'une rotation de l'organe de dosage (3) si bien que l'organe de couplage (2) peut tourner par rapport au boîtier (4) dans une première et de préférence également dans une deuxième direction de rotation.
